# EUROPEAN PATENT SPECIFICATION

(11) **EP 3 691 632 B1**
(45) Date of publication and mention of the grant of the patent: **01.01.2025**
(21) Application number: 18863859.7
(22) Date of filing: 05.10.2018
(51) Int. Cl.: A61K 31/353, A61K 36/82, A61P 35/04, A61K 9/51, A61K 31/495, A61K 31/352

(54) **COMPOSITIONS, METHODS, SYSTEMS AND/OR KITS FOR PREVENTING AND/OR TREATING NEOPLASMS**
ZUSAMMENSETZUNGEN, VERFAHREN, SYSTEME UND/ODER KITS ZUR VORBEUGUNG UND/ODER BEHANDLUNG VON NEOPLASMEN
COMPOSITIONS, MÉTHODES, SYSTÈMES ET/OU KITS DE PRÉVENTION ET/OU DE TRAITEMENT DE NÉOPLASMES

(30) Priority: 06.10.2017 US 201762569413 P
(43) Date of publication of application: 12.08.2020
(73) Proprietor: Research Cancer Institute of America, Fresno, California 93720 (US)
(72) Inventor: NEZAMI, Mohammed, Amin, Fresno CA 93720 (US)
(74) Representative: Zacco Sweden AB
(86) International application number: PCT/US2018/054753
(87) International publication number: WO 2019/071229

(56) References cited:
- WO-A1-2014/091078
- WO-A1-2014/111268
- WO-A2-2007/121088
- US-A1- 2013 011 488
- GAO XIANG ET AL: "Enhancing the anti-colon cancer activity of quercetin by self-assembled micelles", INTERNATIONAL JOURNAL OF NANOMEDICINE, 16 March 2015 (2015-03-16), AUCKLAND, NZ, pages 2051, XP055795401, ISSN: 1176-9114, Retrieved from the Internet <URL:https://doi.org/10.2147/IJN.S75550> [retrieved on 20210415], DOI: 10.2147/IJN.S75550

## Description

### BACKGROUND

### Field

The present disclosure is related to compositions, and/or kits comprising, consisting of, or consisting essentially of, one or more anti-cancer response modulators in combination with one or more anti-cancer agents for preventing and/or treating neoplasms.

Certain embodiments of the present disclosure are related to compositions, and/or kits comprising, consisting of, or consisting essentially of, one or more anti-cancer response modulators in combination with one or more anti-cancer agents for preventing and/or treating neoplasms that are resistant to the one or more anti-cancer agents.

### Description of the Related Art

Although Temodar has been available since the late 1990s and early 2000s, there has been a recent surge in interest in expanding its use owing to its superior ability to penetrate the blood brain barrier. However, the clinical benefits of Temodar have been limited when administered in combination with other drugs.

WO 2014/091078 relates to pharmacology and medicine, and more specifically to slow-release antitumor drug composition based on biodegradable poly(lactic-co-glycolic acid) (PLGA). The composition according to the document comprises temozolomide (TMZ) as the active ingredient and comprises, in addition, surface-active material and a cryoprotectant as parts of nanoparticles.

### SUMMARY

In some embodiments, a pharmaceutical composition for use in prophylaxis, treatment or both of a neoplasm is provided comprising, consisting of, or consisting essentially of, at least one anti-cancer agent, and a first anti-cancer response modulator, wherein the first anti-cancer response modulator is quercetin (QC). In some embodiments of the pharmaceutical composition, the at least one anti-cancer agent is Temodar. In some embodiments of the pharmaceutical composition for use according to the invention, at least a portion of the pharmaceutical composition is formulated for IV administration or oral administration.

In some embodiments of the pharmaceutical composition, the amount of Temodar is about 20 mg to about 5000 mg per day. In some embodiments of the pharmaceutical composition, the amount of quercetin is 0.1 g to 2.5 g. In some embodiments of the pharmaceutical composition, the quercetin is in solution at a concentration of 10 mg/ml to 500 mg/ml. In some embodiments of the pharmaceutical composition for use according to the invention, the anti-cancer agent and at least one anti-cancer response modulator are in a single dosage form for co-administration. In some embodiments of the pharmaceutical composition for use according to the invention, the anti-cancer agent and at least one anti-cancer response modulator are in a single dosage form suitable for IV administration. In some embodiments of the pharmaceutical composition for use according to the invention, the anti-cancer agent and at least one anti-cancer response modulator are in a single dosage form suitable for oral administration. In some embodiments of the pharmaceutical composition for use according to the invention, the anti-cancer agent and at least one anti-cancer response modulator are in a separate dosage forms. In some embodiments of the pharmaceutical composition for use according to the invention, the anti-cancer agent and at least one anti-cancer response modulator are each in dosage forms suitable for IV administration. In some embodiments of the pharmaceutical composition for use according to the invention, the anti-cancer agent and at least one anti-cancer response modulator are each in dosage forms suitable for oral administration. In some embodiments of the pharmaceutical composition for use according to the invention, either the anti-cancer agent or the at least one anti-cancer response modulator is in a dosage form suitable for oral administration and the other is in a dosage form for IV administration.

In some embodiments, the neoplasm is one or more of astrocytomas, pilocytic astrocytoma, diffuse astrocytoma, anaplastic astrocytoma, and brain stem gliomas, glioblastomas, glioblastomas multiforme, meningioma, gliomas, ependymomas, oligodendrogliomas, and mixed gliomas, brain tumors, pituitary tumors, craniopharyngiomas, germ cell tumors, pineal region tumors, medulloblastomas, and primary CNS lymphomas.

In some embodiments, a kit for use in prophylaxis, treatment or both of a neoplasm is provided wherein the kit comprises, consists of, or consists essentially of, a pharmaceutical composition according to any of the claims, wherein the pharmaceutical composition is in a single container. In some embodiments of the kit for use according to the invention, each of the at least one anti-cancer agent and the one or more modulators are contained in a single container in a single dosage form. In some embodiments of the kit for use according to the invention, each of the at least one anti-cancer agent and the one or more modulators are contained in separate sub-containers.

In some embodiments, the pharmaceutical compositions or kits disclosed herein for use in the treatment, prevention or both of a neoplasm in a subject in need thereof is provided. In some embodiments, the neoplasm is likely to develop resistance, develops resistance, and/or is already resistant to the at least one anti-cancer agent. In some embodiments, the neoplasm is likely to develop resistance, develops resistance, and/or is already resistant to the one or more modulators.

In some embodiments, the pharmaceutical composition for use according to the invention is administered to the subject IV, orally or both. In some embodiments, the effect on the neoplasm is an improved result as compared to an effect on the neoplasm of either the at least one anti-cancer agent alone or the one or more modulators alone. In some embodiments, the Temodar is administered at a dose of about 0.0075 mg/m² to about 1000 mg/m² body surface area. In some embodiments, the quercetin is administered at a dose of 0.1 g to 2.5 g. In some embodiments, the SPB is administered at a dose of 0.1 g to 40 g. In some embodiments, the EGCG is administered at a dose is 0.1 g to 1.5 g.

In some embodiments, any of the compositions, kits, uses for use as disclosed herein induces apoptosis in vitro in at least one cancer cell line. In some embodiments, the induction of apoptosis by the composition is additive as compared to the induction of apoptosis of each of the anti-cancer agents and at least one modulators alone. In some embodiments, the induction of apoptosis by the composition is synergistic as compared to the induction of apoptosis of each of the anti-cancer agents and at least one modulator alone.

In some embodiments, a pharmaceutical composition, or kit, for use according to the invention, wherein the modulator is in a nanoparticle formulation. In some embodiments, a pharmaceutical composition, kit, for use according to the invention, the anti-cancer agent is in a nanoparticle formulation. In some embodiments, a pharmaceutical composition, kit, for use accordording to the invention, wherein both the modulator and the anti-cancer agent are in a nanoparticle formulation.

### BRIEF DESCRIPTION OF THE DRAWINGS

FIG. 1 shows size determination of an embodiment of Void-PLGA-PEG-NPs by Dynamic Light Scattering (DLS) (*See,* Example 1).
FIG. 2 shows size determination of an embodiment of FA-PLGA-PEG-NPs (Folic Acid conjugated PLGA-PEG-NPs) by DLS (*See,* Example 1).
FIG. 3 shows size determination of an embodiment of PLGA-PEG-QC NPS (PLGA-PEG NPS encapsulating quercetin) by DLS (*See,* Example 1).
FIG. 4 shows size determination of an embodiment of FA-PLGA-PEG-QC NPS (Folic acid conjugated (PLGAPEG NPS encapsulating quercetin) by DLS (*See,* Example 1).
FIG. 5 shows size determination of an embodiment of DSPE-PEG -NPS (Void-DSPE-PEG/PLGA-PEG-NPS) by DLS
FIG. 6 shows size determination of an embodiment of DSPE-PEG-QC-NPS (DSPE-PEG/PLGA-PEG-NPS encapsulating quercetin) by DLS (*See,* Example 1).
FIG. 7 shows size determination of an embodiment of FA-DSPE-PEG-QC-NPS (Folic acid conjugated DSPEPEG/PLGA-PEG-NPS encapsulating quercetin) by DLS (*See,* Example 1).
FIG. 8 shows shows synthesis of an embodiment of Void-PLGA-PEG nanoparticles using polyvinyl alcohol as a stabilizer (*See,* Example 2).
FIG. 9 shows synthesis of an embodiment of Void-PLGA-PEG nanoparticles using deoxycholic acid as a stabilizer (*See,* Example 3).
FIG. 10 shows synthesis and optimization of an embodiment of PLGA-PEG nanoparticles encapsulating quercetin (*See,* Example 4).
FIG. 11 shows synthesis and optimization of an embodiment of DSPE-PEG-NPs (*See,* Example 5).
FIG. 12 shows effect of an embodiment of nanoformulated quercetin on tumor weight (*See,* Example 9).
FIG. 13 shows effect of a single dose of an embodiment of nanoformulated quercetin on tumor weight (*See,* Example 9).
FIG. 14 shows side effects of an embodiment of the FA-NPS-QC on tumor bearing mice (*See,* Example 9).
FIG. 15 shows an embodiment of a standard curve graph for measurement of quercetin (*See,* Example 9).
FIG. 16 shows an embodiment of a graph of amount of QC in tumor (*See,* Example 9).
FIG. 17 shows an embodiment of a graph of amount of QC in blood plasma (*See,* Example 9).
FIG. 18 shows an embodiment of a Guardant360 Tumor Response Map (*See,* Example 17).
FIG. 19 shows an embodiment of a summary of somatic alterations and associated treatment options (*See,* Example 17).
FIG. 20 shows an embodiment of a graph of the results of the CTC assay (*See,* Example 17).
FIG. 21 shows an embodiment of a graph of serum HER-2 levels before and after treatment (*See,* Example 18).
FIG. 22 shows an embodiment of a graph of serum CA 15-3 levels before and after treatment (*See,* Example 18).
FIG. 23 shows an embodiment of a graph of serum CA 27.29 levels before and after treatment (*See,* Example 18).
FIG. 24 shows an embodiment of a graph of serum lactate dehydrogenase (LDH) levels before and after treatment (*See,* Example 18).
FIG. 25 shows an embodiment of a graph of serum VEGF levels before and after treatment (*See,* Example 19).
FIG. 26 shows an embodiment of a graph of CTC levels before and after treatment (*See,* Example 19).
FIG. 27 shows an embodiment of a graph of serum LDH levels before and after treatment (*See,* Example 19).
FIG. 28 shows an embodiment of a graph of serum CA 15-3 levels before and after treatment (*See,* Example 19).
FIG. 29 shows an embodiment of a graph of serum CA 27.29 levels before and after treatment (*See,* Example 19).
FIG. 30 shows an embodiment of a graph of serum CEA levels before and after treatment (*See,* Example 19).
FIG. 31 shows an embodiment of a graph of serum Her-2 levels before and after treatment (*See,* Example 19).
FIG. 32 shows an embodiment of confocal imaging showing uptake of PLGA-PEG nanoparticles encapsulating Quercetin in cancer cells overexpressing folate receptor (*See,* Example 6).
FIG. 33 shows a data related to tumor weight of in an embodiment of a xenograft study using a chemo-resistant cancer cell line (*See,* Example 8).

### DETAILED DESCRIPTION

Statistics show that deaths caused by advanced cancers of various types have not significantly changed since a decade ago. Indeed, in some cases (e.g., lung cancer) the death rate is rising, especially among women. Even as novel anti-cancer agents are introduced to the market for advanced stages of the disease, patient survival rates have remained essentially unchanged. Moreover, potential toxicity of many novel anti-cancer agents can be a devastating factor both for the clinician and the patient. Additionally, the development of resistance to anti-cancer agents is another cause for concern.

Temodar has been used in three categories in cancer:
(1) Approved indications of Temodar include Advanced Metastatic Melanoma (Orphan), Newly Diagnosed High Grade Glioma (Orphan), Glioblastoma Multiforme, and Anaplastic Astrocytoma. Additional information about approved indications of Temodar is available from the World Wide Web at the URL cancer.gov/about-cancer/treatment/drugs/fda-temozolomide.
(2) Trial or off-label use includes use in other types of cancer, such as sarcomas, non-small cell lung cancers. Additional information about off-label use of Temodar is available from the World Wide Web at the URLs onlinelibrary.wiley.com/doi/10.1002/cncr.11730/pdf, and clinicaltrials.gov/ct2/show/NCT00006877.
(3) An example of Temodar use in solid tumors with CNS metastatic disease is available from the World Wide Web at the URL ncbi.nlm.nih.gov/pubmed/12202665/. In these settings, the highest objective response rate in trials that combined temozolomide (TMZ) with radiotherapy was 0.959, achieved in the study of temozolomide and concurrent radiotherapy in patients with brain metastases from advanced lung cancer and breast cancer.

In March 2005, the U.S. Food and Drug Administration approved temozolomide (TMZ; Temodar^{®} capsules, made by Schering Corporation) for the treatment of adult patients with newly diagnosed glioblastoma multiforme (GBM) concomitantly with (at the same time as) radiotherapy and then as maintenance treatment. GBM is a severe form of brain cancer. In regard to Temodar survival benefit in glioblastoma, addition of Temodar has been reported to only improve survival for 2.5 months in glioblastoma. For example, significantly improved overall survival in patients receiving concomitant and maintenance TMZ + radiation therapy (RT) was observed. The hazard ratio (HR) was 0.63 (95 percent confidence interval for HR=0.52-0.75) with a log-rank p <0.0001 in favor of the combined modality arm. Median survival was 14.6 months (TMZ + RT) versus 12.1 months (RT alone).

Provided herein are compositions, and/or kits comprising, consisting of, or consisting essentially of, one or more anti-cancer response modulators in combination with one or more anti-cancer agents for use in preventing and/or treating neoplasms are provided. In some embodiments, compositions, and/or kits for use according to the invention comprising, consisting of, or consisting essentially of, one or more anti-cancer response modulators in combination with one or more anti-cancer agents for preventing and/or treating neoplasms that are resistant to the one or more anti-cancer agents are provided. In some embodiments, the one or more anti-cancer agents is Temodar. In some embodiments, neoplasms are one or more types of brain cancer.

Also provided herein are embodiments of case studies based on novel combinatorial therapies that provide superior clinical results in a variety of tumor types. In some embodiments, the combinatorial therapies are based on combinations of one or more modulators provided herein and one or more drugs for targeted therapies provided herein.

Thus, present invention relates to a pharmaceutical composition for use in prophylaxis, treatment or both of a neoplasm, wherein the pharmaceutical composition comprises a nanoparticle formulation comprising quercetin, and at least one anti-cancer agent, wherein the at least one anti-cancer agent is Temodar.

Moreover, present invention also relates to a kit for use in prophylaxis, treatment or both of a neoplasm, wherein the kit comprises a pharmaceutical composition according to the invention, wherein each of the at least one anti-cancer agent and quercetin are contained in separate sub-containers.

### Patient population

Provided herein are embodiments of compositions, and/or kits useful for use in preventing and/or treating one or more neoplasms in patients. A neoplasm could be a tumor, a cancer, any new and/or abnormal growth resembling a tumor and/or cancer, or any combination thereof.

In some disclosures, a patient is administered one or more anti-cancer agents to prevent and/or treat a neoplasm. In some embodiments, the patient is naive and never been previously treated with one or more anti-cancer agents. In some cases, the patient may initially respond to the one or more anti-cancer agents resulting in an initial regression of the neoplasm. However, the neoplasm may become resistant to the one or more anti-cancer agents resulting in a relapse. In some embodiments, relapse may also occur due to discontinuing treatment, in which case the relapsed neoplasm may or may not be sensitive to the one or more anti-cancer agents previously administered. Therefore, the patient may either be re-administered the same anti-cancer agent or a different anti-cancer agent. In some embodiments, the patient is initially treated with a first anti-cancer agent, but is subsequently treated with a different second anti-cancer agent. This may be due to several reasons including, but not limited to, development of resistance to the first anti-cancer agent, adverse effects of the first anti-cancer agent, etc.

Therefore, the embodiments of the compositions, and/or kits for use according to the invention provided herein are desirable for patients who are initially responsive but will eventually become non-responsive to one or more anti-cancer agents, or in patients who were initially responsive but have now become non-responsive to one or more anti-cancer agents. The embodiments are also desirable for patients who are non-responsive because they have a neoplasm that is resistant to one or more anti-cancer agents.

In some embodiments, the patient is a male or a female. A patient is typically human but animals other than human are also contemplated. Non-limiting examples of the animals other than human include without limitation domestic animals, pets, experimental animals, and/or commercially important animals.

### Tumor and/or cancer types

Disclosed herein are examples of neoplasms, which could be a tumor, a cancer, any new and/or abnormal growth resembling a tumor and/or cancer, or any combination thereof. In some embodiments, the neoplasm is a breast carcinoma or a breast adenocarcinoma, or any neoplasm associated with breast. In some embodiments, neoplasm is a non-small cell lung carcinoma or lung adenocarcinoma, or any neoplasm associated with lung. In some embodiments, the neoplasm is a uterine sarcoma, or any neoplasm associated with uterus. In some embodiments, the neoplasm is a pancreatic adenocarcinoma, or any neoplasm associated with pancreas. In some embodiments, the neoplasm is a malignant melanoma, or any neoplasm associated with skin.

In some embodiments, the neoplasm is a glioblastoma, or any neoplasm associated with brain. In some embodiments, the neoplasm is related to one or more types of neoplasm provided herein.

In some embodiments, the neoplasm is a glioblastoma, or any neoplasm associated with brain. In some embodiments, the neoplasm is one or more types of brain cancer. Non-limiting examples of one or more types of brain cancer include astrocytomas (e.g., pilocytic astrocytoma, diffuse astrocytoma, anaplastic astrocytoma, and brain stem gliomas), glioblastomas (e.g., glioblastomas multiforme), meningioma, other gliomas (e.g., ependymomas, oligodendrogliomas, and mixed gliomas), and other brain tumors (e.g., pituitary tumors, craniopharyngiomas, germ cell tumors, pineal region tumors, medulloblastomas, and primary CNS lymphomas). *See,* cancercenter.com/brain-cancer/types/tab/overview/. In some embodiments, the neoplasm is related to one or more types of neoplasm provided herein.

In some embodiments, the neoplasm is likely to become resistant and/or is already resistant to one or more anti-cancer agents. Thus, the embodiments provided herein are particularly useful for use in preventing and/or treating neoplasm that are resistant to or are likely to become resistant to one or more anti-cancer agents. In some embodiments, the neoplasm is not resistant and/or is not likely to become resistant to one or more anti-cancer agents. Thus, the embodiments provided herein are useful for preventing and/or treating neoplasm that is not resistant and/or is not likely to become resistant to one or more anti-cancer agents by administering a minimum dose of one or more anti-cancer agents sufficient to prevent and/or treat the neoplasm.

Examples of other neoplasms include breast adenocarcinoma, pancreatic adenocarcinoma, lung carcinoma, prostate cancer, glioblastoma multiform, hormone refractory prostate cancer, solid tumor malignancies such as colon carcinoma, non-small cell lung cancer (NSCLC), anaplastic astrocytoma, bladder carcinoma, sarcoma, ovarian carcinoma, rectal hemangiopericytoma, pancreatic carcinoma, advanced cancer, cancer of large bowel, stomach, pancreas, ovaries, melanoma pancreatic cancer, colon cancer, bladder cancer, hematological malignancies, squamous cell carcinomas, and breast cancer.

In some embodiments, the neoplasm is one or more of breast adenocarcinoma, pancreatic adenocarcinoma, lung carcinoma, prostate cancer, glioblastoma multiform, hormone refractory prostate cancer, solid tumor malignancies such as colon carcinoma, non-small cell lung cancer (NSCLC), anaplastic astrocytoma, bladder carcinoma, sarcoma, ovarian carcinoma, rectal hemangiopericytoma, pancreatic carcinoma, advanced cancer, cancer of large bowel, stomach, pancreas, ovaries, melanoma pancreatic cancer, colon cancer, bladder cancer, hematological malignancies, squamous cell carcinomas, breast cancer, astrocytomas, pilocytic astrocytoma, diffuse astrocytoma, anaplastic astrocytoma, and brain stem gliomas, glioblastomas, glioblastoma multiforme, meningioma, gliomas, ependymomas, oligodendrogliomas, and mixed gliomas, brain tumors, pituitary tumors, craniopharyngiomas, germ cell tumors, pineal region tumors, medulloblastomas, and primary CNS lymphomas.

### Anti-cancer agents

As used herein an "anti-cancer agent" can be an anti-cancer agent, anti-tumor agent, anti-cancer drug and/or anti-tumor drug that slows the growth, stops the growth, causes a reduction in size, eliminates and/or prevents relapse of a neoplasm.

In some embodiments, the anti-cancer agents are well-known in the art and in some embodiments are approved for therapeutic use and/or use in clinical trials by government agencies (e.g., FDA, EMEA, etc.). The dosing, route of administration, efficacy against known neoplasm types, side/adverse effects, mechanism of action, etc. of the anti-cancer agents may also be well-known in the art. In other embodiments, the anti-cancer agent is a compound that is believed to have anti-cancer effects (e.g., without being limiting, in vitro, in vivo and/or ex vivo in a laboratory and/or in a human clinical trial), but is not yet approved by a government agency for the treatment of cancer. An example of an anti-cancer agent includes Temodar.

### Anti-cancer response modulators

As used herein an "anti-cancer response modulator" (also referred to herein as "modulator") improves the anti-cancer effect of a known or a novel anti-cancer agent against a neoplasm when used in combination with one or more of the modulators disclosed herein. In some embodiments, the anti-cancer agent may not have any effect in the absence of the modulator. In some embodiments the anti-cancer agent improves the anti-cancer activity of the modulator. In some embodiments, the modulator improves the anti-cancer activity of the anti-cancer agent. In some cases, the two work in concert to improve the anti-cancer activity of each other. In some embodiments, the neoplasm is likely to develop resistance, develops resistance, and/or is already resistant to the known or a novel anti-cancer agent. In some embodiments, the neoplasm is likely to develop resistance, develops resistance, and/or is already resistant to one or more modulators.

Several embodiments of modulators are contemplated. Examples of modulators include those that improve the effect of anti-cancer agents including, but not limited to, anti-cancer agents that are effective against one or more types of neoplasm but ineffective against one or more other types of neoplasm.

Thus, modulators improve the effect of anti-cancer agents in a patient who may be non-responsive to a particular anti-cancer agent or the anti-cancer agent may be ineffective in a patient with a particular type of neoplasm even before initiation of treatment with the anti-cancer agent. The modulator can improve the effect of anti-cancer agents in a patient who may initially be responsive to a particular anti-cancer agent or the anti-cancer agent may initially be effective in a patient with a particular type of neoplasm but may eventually become ineffective. In some embodiments, the modulator can improve the effect of anti-cancer agents in a patient with a relapse of the neoplasm.

An exampls of a modulators include quercein. Quercetin is a flavonol found in many fruits, vegetables, leaves, and grains. It can be used as an ingredient in supplements, beverages, or foods. Quercetin is one of the most abundant dietary flavonoids with an average daily consumption of 25-50 mg. The modulators provided herein are non-toxic and/or minimally toxic with no and/or minimal side effects.

### Dose of modulator

In some embodiments, quercetin for use according to the invention is administered intravenously. The concentration of quercetin in a solution for intravenous administration is about 5 mg/ml to about 500 mg/ml. In some embodiments, the concentration of quercetin in a solution for intravenous administration is about 50 mg/ml. In some embodiments, quercetin is administered intravenously at a dose of about 0.05 g to about 10 g. In some embodiments, quercetin is administered intravenously at a dose of about 0.5 g to about 1 g. In some embodiments, quercetin is administered intravenously at a dose of about 0.05, 0.1, 0.2, 0.3, 0.4, 0.5, 0.6, 0.7, 0.8, 0.9, 1, 1.5, 2, 2.5 3, 3.5, 4, 4.5, 5, 5.5, 6, 6.5, 7, 7.5, 8, 8.5, 9, 9.5 or 10 g, or within a range defined by any two of the aforementioned values.

In some embodiments, quercetin for use according to the invention is administered orally. The amount of quercetin in a composition for oral administration is about 100 mg to about 10 g. In some embodiments, quercetin is administered orally at a dose of about 0.5 g to about 4 g. In some embodiments, quercetin is administered orally at a dose of about 1 g. In some embodiments, quercetin is administered orally at a dose of about 0.05, 0.1, 0.2, 0.3, 0.4, 0.5, 0.6, 0.7, 0.8, 0.9, 1, 1.5, 2, 2.5 3, 3.5, 4, 4.5, 5, 5.5, 6, 6.5, 7, 7.5, 8, 8.5, 9, 9.5, 10, 15, 20, 25, 30, 35, 40, 45 or 50 g, or within a range defined by any two of the aforementioned values. In some embodiments, the composition for use according to the invention for oral administration can be prepared into a solution for intravenous administration, wherein the concentration of quercetin is about 10 mg/ml to about 100 mg/ml. In some embodiments, quercetin for use according to the invention is administered in a liposomal formulation. In some embodiments, quercetin for use according to the invention is administered in a liposomal formulation at 50 mg a day. In some embodiments, quercetin for use according to the invention is administered in a liposomal formulation at about 25 mg a day to about 75 mg a day. In some embodiments, quercetin for use according to the invention is administered in a liposomal formulation at about 10, 15, 20, 25, 30, 35, 40, 45, 50, 55, 60, 65, 70, 75, 80, 85, 90, 95 or 100 mg a day, or within a range defined by any two of the aforementioned values.

In some embodiments, Quercetin by itself is not water soluble and therefore is administered in the oral dosage form. In some embodiments, Quercetin is available in the form of propylene glycol-Quercetin (Quercetin PG) and propylene ethylene glycol-Quercetin (Quercetin PEG). Quercetin PG is water soluble and is used in the clinic as the IV dosage form.

### Combinations

A surprising and unexpected anti-cancer effect was observed when one or more anti-cancer agents were used in combination with one or more modulators provided herein. The surprising and unexpected result was a better than expected result wherein the effectiveness of the one or more anti-cancer agents was improved when used in combination with one or more modulators as compared to the anti-cancer agent in the absence of the modulator(s). The potentiation was achieved by co-administering the one or more anti-cancer agents and one or more modulators.

Therefore, provided herein are combinations of one or more anti-cancer agents and one or more modulators for use according to the invention. The one or more modulators can improve the effect of one or more anti-cancer agent against one or more neoplasm types provided herein. The potentiation can occur in several ways. Non-limiting examples include enhancing the effectiveness of an already effective anti-cancer agent, making an ineffective anticancer agent effective (the anticancer agent could also have been previously effective but become ineffective following long term and/or short term use in a patient), increasing the length of time for which an anti-cancer agent is effective, decreasing the effective dose of administration of the anti-cancer agent, decreasing the duration of time for which anti-cancer agent is administered, decreasing the frequency of administration of an anti-cancer agent, and/or enabling the administration of anti-cancer agent via a more amenable route.

The one or more anti-cancer agents for use according to the invention can be provided at any dose, via any of the routes of administration, in any order of administration, at any frequency of administration, and/or any dosage form provided herein. Similarly, the one or more modulators for use according to the invention can be provided at any dose, via any of the routes of administration, in any order of administration, at any frequency of administration, and/or any dosage form provided herein. Also, the combination of one or more anti-cancer agent and one or more modulators for use according to the invention can be provided at any dose, via any of the routes of administration, in any order of administration, at any frequency of administration, and/or any dosage form provided herein.

Combinations can comprise, consist of, consist essentially of, one or more anti-cancer agents and one or more modulators. In some embodiments, the combination comprises, consists of, or consists essentially of, one or more anti-cancer agents and one modulator selected from quercetin. In some embodiments, the combination comprises, consists of, or consists essentially of, one or more anti-cancer agents and at least one modulator. In some embodiments, the at least one modulator is selected from quercetin. For example, in some embodiments, the combination can comprise, consist of, consist essentially of, the anticancer agent and quercetin. In some embodiments, the combination comprises, consists of, or consists essentially of, one or more anti-cancer agents and a modulator. In some embodiments, the anti-cancer agent is Temodar.

The potentiation can be additive or synergistic. A synergistic effect is greater than an additive effect. An additive effect is observed when the potentiation is equal to the sum of the individual effects of the anti-cancer agent(s) and modulator. A synergistic effect is observed when the potentiation is greater than the sum of the individual effects of the anti-cancer agent and modulator. Synergistic effect, additive effect or both can occur in human patients, non-human patients, non-patient human volunteers, in vivo models, ex vivo models, in vitro models, etc.

Potentiation can range from about <1 to about 100 fold. In some embodiments, the synergistic effect is about 3 to about 30 fold. In some embodiments, the potentiation ranges from <1, 1, >1, 2, 3, 4, 5, 6, 7, 8, 9, 10, 20, 30, 40, 50, 60, 70, 80, 90, or 100 fold, or within a range defined by any two of the aforementioned values.

In some embodiments, a synergistic effect allows for a reduction in the requirement of an anti-cancer agent to about 25% to about 75% of the recommended dose. In some embodiments, a synergistic effect allows for a reduction in the requirement of an anti-cancer agent to about 5, 10, 20, 30, 40, 50, 60, 70, 80, 90, or 95% of the recommended dose, or a value within a range defined by any two of the aforementioned values.

For example, the combination of Temodar with quercetin can produce a synergistic effect on a neoplasm as compared to Temodar alone.

According to the disclosure, an additive and/or synergistic or sustained response to a combinational therapy is observed. Thus, "combination therapy" is intended to encompass administration of these therapeutic agents in a sequential manner, wherein each therapeutic agent is administered at a different time, as well as administration of these therapeutic agents, or at least two of therapeutic agents concurrently, or in a substantially simultaneous manner. Simultaneous administration can be accomplished, for example, by administering to the subject a single dosage form, for example, a solution, pill or capsule, having a fixed ratio of each therapeutic agent or in multiple, single dosage forms for each of therapeutic agents. Sequential or substantially simultaneous administration of each therapeutic agent can be effected by any appropriate route including, but not limited to, oral routes, intravenous routes, intramuscular routes, and direct absorption through mucous membrane tissues.

According to the disclosure, mixtures of compositions of the present invention can also be administered to the patient as a simple mixture or in suitable formulated pharmaceutical compositions. Thus, the combination therapy can be achieved by administering two or more agents, e.g., two or more other therapeutic agents, each of which is formulated and administered separately, or by administering two or more agents in a single formulation. Other combinations are also encompassed by combination therapy. For example, two agents can be formulated together and administered in conjunction with a separate formulation containing a third agent. While the two or more agents in the combination therapy can be administered simultaneously, they need not be. For example, administration of a first agent (or combination of agents) can precede administration of a second agent (or combination of agents) by minutes, hours, days, or weeks. Thus, the two or more agents can be administered within minutes of each other or within 1, 2, 3, 6, 9, 12, 15, 18, or 24 hours of each other or within 1, 2, 3, 4, 5, 6, 7, 8, 9, 10, 12, 14 days of each other or within 2, 3, 4, 5, 6, 7, 8, 9, or 10 weeks of each other. In some cases even longer intervals are possible. While in many cases it is desirable that the two or more agents used in a combination therapy be present in within the patient's body at the same time, this need not be so in other cases.

The potentiation can be measured in one or more assays that measure effects such as apoptosis, cellular metabolic changes, cellular morphological changes, etc., or other effects that would be well-known to one of ordinary skill in the art. In some embodiments, the combination of anti-cancer agent(s) and one or more modulators causes an induction of apoptosis, which can be measured using the MiCK^{®} assay (Example 20). In some embodiments, the modulator can suppress angiogenesis within/around the neoplasm. For example, Quercetin can cause suppression of angiogenesis in glioma cells.

According to the disclosure, the modulator provided herein is effective as an anticancer agent without the presence of an anti-cancer agent. Thus, the modulator provided herein is independently effective as anti-cancer agents, i.e., without co-administration of one or more anti-cancer agents. For example, an anti-cancer effect is observed when quercetin administered without co-administration of one or more anti-cancer agents (*See,* Example 15 - Example 19). (*See,* Example 15, and Example 17 - Example 19).

However, when the modulator provided herein is administered in combination with one or more anti-cancer agents, a synergistic anti-cancer effect is observed. Thus, in some embodiments, a synergistic anti-cancer effect is observed when the modulator provided herein is co-administered with the one or more anti-cancer agents provided herein. For example, a synergistic anti-cancer effect is observed when Quercetin-PG is co-administered with Temodar (*See,* Example 15 - Example 19).

### Route of administration according to the disclosure

According to the disclosure, the route of administration of the modulator(s) and anti-cancer agent(s) can be determined by one of ordinary skill in the art based on the circumstances. Several routes of administrations are possible including parenteral, subcutaneous, intrarticular, intrabronchial, intraabdominal, intracapsular, intracartilaginous, intracavitary, intracelial, intracelebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, intralesional, bolus, vaginal, rectal, buccal, sublingual, intranasal, or transdermal.

Any route of administration provided herein can be used for the combination of anti-cancer agent(s) and modulator or for the individual components of the combination. For example, the combination of anti-cancer agent and modulator can be administered intravenously, orally or both. In some embodiments, one or more components in the combination can be administered via one route (e.g., intravenously) and the other components can be administered via a different route (e.g., orally). In some embodiments, all components in the combination are administered via the same route (e.g., either intravenously or orally). The anticancer agent and modulators can be administered via any combination of intravenous and oral routes as shown in Table 1.

**Table 1 - Combinations comprising, consisting of, or consisting essentially of, one anti-cancer agent and one modulators (S1).**

| | **Route of administration** | | |
|---|---|---|---|
| **Combination** | **Anti-cancer agent** | **S1** | |
| 1 | IV | IV | |
| 2 | Oral | IV | |
| 3 | IV | Oral | |
| 4 | IV | IV | |
| 5 | Oral | Oral | |
| 6 | Oral | IV | |
| 7 | IV | Oral | |
| 8 | Oral | Oral | |

| | | | |
|---|---|---|---|
| In Table 1, the anti-cancer agent is Temodar, S 1 is quercetin. | | | |

### Order of administration according to the disclosure

Any order of administration can be used for the one or more anti-cancer agents and one or more modulators in a combination. For example, the one or more anti-cancer agents and the one or more modulators in the combination can be administered simultaneously or sequentially. For example, all components of the combination are administered simultaneously, or only some of the components of the combination are administered simultaneously and the rest are administered sequentially. Alternatively, none of the components are administered simultaneously, i.e., all the components are administered sequentially. When administering sequentially, any order of administration can be used. For example, when administering a combination of one anti-cancer agent and two modulators, the anti-cancer agent can be administered first followed by the two modulators either simultaneously or sequentially in any order, or the two modulators can be administered first either simultaneously or sequentially in any order followed by the anti-cancer agent, or one of the modulator each can be administered before and after the administration of the anti-cancer agent. Additional orders of administration are possible and contemplated when the combination comprises, consists of, or consists essentially of, additional components, for example, a third modulator.

### Frequency of administration according to the disclosure

Frequency of administration of the anti-cancer agent is as known in the art. Frequency of administration of the anti-cancer agent can be varied depending various parameters such as level of potentiation, prognosis following administration of a combination provided herein, patient compliance, side effects, etc., for example, daily, weekly, biweekly, monthly, bimonthly, or as is known in the art. Modulators can be administered along with the anti-cancer agent daily, weekly, biweekly, monthly, bimonthly, less frequently compared to the anti-cancer agent, or more frequently compared to the anti-cancer agent.

Administration can be daily, or 1, 2, 3, 4, 5, 6 or more times weekly, or more or less frequently as required. Administration can be provided as a single dose or as divided doses, such that a daily dose may be given in 2, 3, 4, or more portions in a single day.

Co-administration of the components of a combination may comprise, consist of, consist essentially of, administering the components simultaneously, or within about 1, 5, 15, 30, 45 or 60 minute of one another, or within any range defined by the aforementioned values. Co-administration of the components of a combination may comprise, consist of, consist essentially of, administering the components within about 1 hour to within about 6 hours of one another, or within any range defined by the aforementioned values.

### Pharmaceutical formulations for use according to the invention

In some embodiments, pharmaceutical formulations for use in prophylaxis, treatment or both of a neoplasm are provided. The formulation can be a single composition for co-administration comprising, consisting of, or consisting essentially of, at least one anti-cancer agent and one, two, three or more modulators. In some embodiments, the formulation comprises, consists of, or consists essentially of, more than one composition, e.g. the anti-cancer agent in one dosage form, and the one or more modulators in a second, third or fourth dosage form. Several compositions are contemplated. The type of composition to be administered can be determined by one of ordinary skill in the art based on the circumstances under which administration is desired.

The compositions provided herein comprise, consist of, consist essentially of, active ingredients, inactive ingredients, excipients, additives, and/or pharmaceutically acceptable carriers. Examples of additives include natural polymer compounds, inorganic salts, binders, lubricants, disintegrants, surfactants, thickeners, coating agents, pH adjusters, antioxidants, flavoring agents, preservatives, and colorants among others. Examples of other pharmaceutically acceptable carriers include liquid carriers such as water, alcohol, emulsion, and solid carriers such as gel, powder, etc. Standard pharmaceutical formulation techniques and ingredients can be used, such as those disclosed in Remington's The Science and Practice of Pharmacy, 21st Ed., Lippincott Williams & Wilkins (2005).

Compositions for intravenous administration comprise, consist of, consist essentially of, excipient and pharmaceutically acceptable carries including one or more of sodium chloride, dextrose, and sterile water. Compositions can comprise, consist of, consist essentially of, aqueous isotonic sterile injection solutions, which can comprise, consist of, consist essentially of, one or more of antioxidants, buffers, bacteriostats, and solutes that render the formulation isotonic with the blood of the intended recipient, and aqueous and nonaqueous sterile suspensions that can include suspending agents, solubilizers, thickening agents, stabilizers, and preservatives.

Compositions for oral administration can be any dosage form that is suitable for oral ingestion, for example, liquid compositions such as elixir, suspension, syrup, emulsion, ampoule, etc., solid compositions such as gel, gum, drop, powder, granule, pill, sugar-coated tablet, film-coated tablet, capsule, package agent, etc. Also contemplated are sustained-release compositions such as gel-coated compositions, multi-coated compositions, localized release compositions.

In some embodiments, the compositions for use according to the invention are administered by intravenous infusion. The compositions can be presented in unit-dose or multi-dose sealed containers, such as ampules and/or vials. Injection solutions and suspensions can be prepared from sterile powders, granules, and/or tablets. In some embodiments, the compositions to be administered can be formulated as pharmaceutical formulations for delivery via one or more of the routes provided herein.

A composition for use according to the invention can comprise, consist of, consist essentially of, a combination of one or more anti-cancer agents and one or more modulators, wherein the one or more anti-cancer agents and one or more modulators can be present in any dosage form. For example, in a composition comprising, consisting of, or consisting essentially of, a combination of one anti-cancer agent and two modulators, all three components can be in the same dosage form (e.g., for intravenous administration or for oral administration), or one of the components in the combination can be of one dosage form (e.g., for intravenous administration or for oral administration) and other two component in the combination can be of a different dosage form (e.g., for intravenous administration or for oral administration). In some embodiments, all three components in the combination may be of a different dosage form (e.g., for intravenous administration, for oral administration, and a third dosage form).

For example, in a composition comprising, consisting of, or consisting essentially of, a combination of Temodar as the anti-cancer agent and quercetin as the modulator, all can be in an intravenous or oral dosage form, Temodar can be in an intravenous dosage form and quercetin can be in an oral dosage form, or Temodar can be in oral dosage form and quercetin can be in an intravenous dosage form.

### Kits

Inasmuch as it may desirable to administer a combination provided herein, for example, for the purpose of preventing and/or treating a neoplasm, it is within the scope of the present disclosure to provide the components of a combination as a kit such that the components of the combination are suitable for co-administration.

A kit comprising, consisting of, or consisting essentially of, one or more anti-cancer agents to be used in combination with a modulator is provided.

The components can be separately provided such as in separate containers, or in separate compartments of a divided bottle or divided foil packet (e.g., a blister pack used for the packaging of tablets, capsules, etc.).

The kit is particularly suitable for administering different dosage forms, for example, oral and intravenous, for administering the components at different dosage intervals, and/or for titration of components against one another. The kit typically comprises, consists of, or consists essentially of, directions for administration and may additionally be provided with a memory aid to ensure compliance.

The components in the kit may exist in dissolved form, undissolved form or a combination thereof. If present in undissolved form, the undissolved component may be combined with another component present in a dissolved form in a specific stoichiometric amount prior to use. If all the components are present in an undissolved form, the components can either be administered as such (e.g., orally) or dissolved into a solvent (e.g., water) prior to administration (e.g., intravenously).

In some embodiments, the kit comprises, consists of, or consists essentially of, Temodar as the anti-cancer agent to be used in combination with quercetin a as the modulator.

### Temodar

Temozolomide (TMZ; brand names Temodar, Temodal and Temcad) is an oral chemotherapy drug. It is an alkylating agent used for treating certain brain cancers. It is used as a first-line treatment for glioblastoma multiforme and as a second-line treatment for astrocytoma.

In some embodiments, the amount of Temodar ranges between about 20 mg and about 2000 mg per day. In some embodiments, the amount of Temodar ranges between about 8 mg and about 5000 mg per day. In some embodiments, the amount of Temodar is about 8, 8.5, 9, 9.5, 10, 25, 50, 75, 100, 250, 500, 750, 1000, 1500, 2000, 2500, 3000, 3500, 4000, 4500, or 5000 mg per day, or within a range defined by any two of the aforementioned values. In some embodiments the dose of Temodar is about 0.0075 mg/m² to about 20 mg/m². In some embodiments the dose of Temodar is about 0.0015 mg/m² to about 1000 mg/m². In some embodiments the dose of Temodar is about 0.0015, 0.003, 0.006, 0.012, 0.024, 0.036, 0.048, 0.060, 0.072, 0.084, 0.096, 0.2, 0.25, 0.5, 0.75, 1, 2, 3, 6, 12, 18, 24, 30, 40, 50, 60, 70, 80, 90, 100, 200, 300, 400, 500, 600, 700, 800, 900, or 1000 mg/m², or within a range defined by any two of the aforementioned values.

Other regimens of intravenous and oral Temodar have also been reported. Dosages must be adjusted in accord with objective indicator for regulating dosage, for example, evidence of antitumor activity, leukopenia or both. Total leukocyte count is a good, objective indicator for regulating dosage.

Temodar is effective alone for susceptible malignancies. However, Temodar can be used concurrently or sequentially with one or more other antineoplastic drugs. When Temodar is included in combined cytotoxic regimens, the doses of Temodar as well as that of the other drugs are adjusted accordingly as known to those of skill in the art.

The compositions, and/or kits provided herein can be for use in treating neoplasms. In some embodiments, the neoplasm is one or more types of brain cancer. Examples of one or more types of brain cancer include glioblastoma, or any neoplasm associated with brain, astrocytomas (e.g., pilocytic astrocytoma, diffuse astrocytoma, anaplastic astrocytoma, and brain stem gliomas), glioblastomas (e.g., glioblastomas multiforme), meningioma, other gliomas (e.g., ependymomas, oligodendrogliomas, and mixed gliomas), and other brain tumors (e.g., pituitary tumors, craniopharyngiomas, germ cell tumors, pineal region tumors, medulloblastomas, and primary CNS lymphomas). *See,* cancercenter.com/brain-cancer/types/tab/overview/. In some embodiments, the neoplasms are sarcomas, non-small cell lung cancers, solid tumors with CNS metastatic disease. In some embodiments, the neoplasms are related to one or more types of neoplasm provided herein. -cell) leukemia, mycosis fungoides (advanced disease), neuroblastoma (disseminated disease), adenocarcinoma of the ovary, retinoblastoma, carcinoma of the breast, triple negative breast cancer, and biopsy-proven minimal change nephrotic syndrome in pediatrics patients who failed to adequately respond to or are unable to tolerate adrenocorticosteroid therapy.

In one embodiment, the combination comprises, consists of, or consists essentially of, Temodar and quercetin.

In some embodiments, the amount of Temodar ranges between about 20 mg and about 2000 mg per day. In some embodiments, the amount of Temodar ranges between about 8 mg and about 5000 mg per day. In some embodiments, the amount of Temodar is about 8, 8.5, 9, 9.5, 10, 25, 50, 75, 100, 250, 500, 750, 1000, 1500, 2000, 2500, 3000, 3500, 4000, 4500, or 5000 mg per day, or within a range defined by any two of the aforementioned values. In some embodiments the dose of Temodar is about 0.0075 mg/m² to about 20 mg/m². In some embodiments the dose of Temodar is about 0.0015 mg/m² to about 1000 mg/m². In some embodiments the dose of Temodar is about 0.0015, 0.003, 0.006, 0.012, 0.024, 0.036, 0.048, 0.060, 0.072, 0.084, 0.096, 0.2, 0.25, 0.5, 0.75, 1, 2, 3, 6, 12, 18, 24, 30, 40, 50, 60, 70, 80, 90, 100, 200, 300, 400, 500, 600, 700, 800, 900, or 1000 mg/m², or within a range defined by any two of the aforementioned values.

Doses of quercetin for intravenous administration can be any one of the doses provided herein. For example, doses of quercetin for intravenous administration include 0.5 g to 1 g.

In some embodiments, the combinations herein are provided as compositions, and/or kits for use in preventing neoplasms in patients and/or for use in treating neoplasms in patients. Any of the doses, routes of administration, frequency of administration, sequence of administration, etc. provided herein can be used for the components of the combinations.

The dose, route of administrations, mechanism of action, etc. of Temodar are well-known in the art. The dose of Temodar can be varied depending on, among other aspects, patient age, route of administration, neoplasm type, etc.

### Nanoparticle formulations

In some embodiments, the pharmaceutical formulation for use according to the invention is formulated as one or more of a nanoparticle formulation, a liposomal formulation, or folic acid receptor conjugates. Nanoparticle formulations have many advantages over traditional dosage forms, such as enhanced dissolution properties and potential for efficient intracellular delivery of drugs. Nanoparticles have unique physical and chemical properties that offer several advantages as drug delivery carriers, or 'nano-carriers.' Nanoparticles-based composition for detection and/or treatment of cancers can comprise, consist of, consist essentially of, nanoparticles in the form of, without limitations, quantum dots, magnetic nanoparticles, gold nanoshells (which are useful in detecting tumors and metastasis in many solid tumors), poly (lactide-co-glycolide) (PLGA)-based nanoparticles (e.g., PLGA/montmorillonite (PLGA/MMT) nanoparticles, Vitamin E-TPGS-emulsified PLGA nanoparticles, PLGA-mPEG nanoparticles), dendrimers, and SPIO and USPIO nanoparticles. *See,* Mousa S.A. and Bharali D.J., Nanotechnology-Based Detection and Targeted Therapy in Cancer: Nano-Bio Paradigms and Applications, Cancers (Basel), Vol. 3, No. 3, pp. 2888-2903, September 2011. Other nanoparticle-based examples are provided in Bharali D.J. and Mousa S.A., Emerging nanomedicines for early cancer detection and improved treatment: current perspective and future promise, Pharmacology & Therapeutics, Vol. 128, No. 2, pp. 324-335, Nov 2010, and Bharali D.J., et al., Nanoparticles and cancer therapy: a concise review with emphasis on dendrimers, International Journal of Nanomedicine, Vol. 4, pp. 1-7, April 1, 2009. Pharmaceutical compositions can also be formulated as nano-micelles-based compositions, for example, as provided in US 9,308,270 B2ty. Folic acid receptor conjugates based on a conjugating a molecule/drug with folic acid to form a "folate conjugate." Owing to the naturally high affinity of folate for the folate receptor protein commonly expressed on the surface of many human cancers, folate conjugates bind tightly to the folate receptor protein and trigger cellular uptake via endocytosis. Diverse molecules/drugs can be successfully delivered inside folate receptor protein expressing cells and tissues. Liposomal formulations comprise, consist of, consist essentially of, liposomes that are used as vehicles for administration of drugs. Liposomes are most often composed of phospholipids, especially phosphatidylcholine, but may also include any lipids that are compatible with a lipid bilayer structure (e.g., as egg phosphatidylethanolamine). A liposomal formulation can comprise, consist of, consist essentially of, liposomes that may employ surface ligands for attaching to unhealthy tissue. The major types of liposomes are multilamellar vesicle with several lamellar phase lipid bilayers, small unilamellar liposome vesicle with one lipid bilayer, large unilamellar vesicle, and cochleate vesicle.

In some embodiments, nanoparticle formulations are provided. The nanoparticle formulations comprise, consist of, consist essentially of, one or more anti-cancer agents and/or one or more modulators, wherein the one or more anti-cancer agents and/or one or more modulators can be present in any dosage form, for example liquids for injection or oral administration, or pills or capsules. In some embodiments, the nanoparticles contain a single compound, i.e., one anti-cancer agent or one modulator (e.g., Temodar, and quercetin).

In some embodiments, nanoparticle formulations comprise, consist of, consist essentially of, at least one compound as nanoparticles. For example, in some embodiments, when nanoparticle formulations comprise, consist of, consist essentially of, two compounds (i.e. Temodar + quercetin, wherein at least one compound is present as nanoparticles. In some embodiments, when nanoparticle formulations comprise, consist of, consist essentially of, two compounds (i.e. Temodar + quercetin, both compounds are present as nanoparticles. In some embodiments, nanoparticle formulations comprise, consist of, consist essentially of, nanoparticles containing a single compound, i.e., one anti-cancer agent or one modulator (i.e. nano-Temodar, nano-quercetin. In some embodiments, nanoparticle formulations comprise, consist of, consist essentially of, nanoparticles containing two compounds (i.e. nano-Temodar + nano-quercetin.

In some embodiments, provided herein are nanoparticle formulations for use in preventing and/or treating one or more neoplasms. In some embodiments, the one or more neoplasm is one or more types of brain cancer. Non-limiting examples of one or more types of brain cancer include astrocytomas (e.g., pilocytic astrocytoma, diffuse astrocytoma, anaplastic astrocytoma, and brain stem gliomas), glioblastomas (e.g., glioblastomas multiforme), meningioma, other gliomas (e.g., ependymomas, oligodendrogliomas, and mixed gliomas), and other brain tumors (e.g., pituitary tumors, craniopharyngiomas, germ cell tumors, pineal region tumors, medulloblastomas, and primary CNS lymphomas). *See,* cancercenter.com/brain-cancer/types/tab/overview.

In some embodiments, nanoparticles comprise, consist of, consist essentially of quercetin, and a chemotherapeutic agent. In some embodiments, nanoparticle formulations comprise, consist of, consist essentially of, a combination of nanoparticles wherein a first population of nanoparticles contains quercetin, and a second population of nanoparticles contains a chemotherapeutic agent. In some embodiments, the chemotherapeutic agent is Temodar.

In some embodiments, the nanoparticle formulation comprises, consists of, or consists essentially of, Temodar + quercetin in a nanoparticle, or a combination of a first population of nanoparticles containing Temodar and a second population of nanoparticles containing quercetin. It is believed that the nanoparticles are expected to improve the ability of a compound to cross the blood brain barrier. For example, in a nanoparticle formulation comprising, consisting of, or consisting essentially of, a combination of Temodar + quercetin in a single nanoparticle or in two separate nanoparticle populations, it is expected that the nanoparticles will improve the ability of Temodar and quercetinto cross the blood brain barrier.

In some embodiments, the nanoparticles can be one or more nanospheres, nanocylinders, nanoplates, nanoshells, nanorods, nanorices, nanofibers, nanowires, nanopyramids, nanoprisms, nanostars, nanocrescents, nanorings, and nanoantennas. In some embodiments, the dimensions of the nanoparticles can range from about 1 nm to about 100 nm. In some embodiments, the dimensions of the nanoparticles can range from about 100 nm to about 250 nm. In some embodiments, the dimensions of the nanoparticles can range from about 20 nm to about 1000 nm. In some embodiments, the dimensions of the nanoparticles can range from about 4 nm to about 6250 nm. In some embodiments, the dimensions of the nanoparticles is about 1, 5, 10, 20, 30, 40, 50, 60, 70, 80, 90, 100, 200, 300, 400, 500, 750, 1000, 1250, 2500, 3750, 5000, 7500, 10000 nm, or a value within a range defined by any two of the aforementioned values. In some embodiments, the amount of modulator and/or the amount of anti-cancer agent that can be incorporated within a nanoparticle depends on the size of the nanoparticle. Thus, the greater the size of a nanoparticle, the greater the amount of one or more modulators and/or anti-cancer agent that can be incorporated within the nanoparticle.

In some embodiments, the nanoparticles comprise, consist of, consist essentially of, one or more stabilizers, which comprise, consist of, consist essentially of, the structural components of the nanoparticle. Non-limiting examples of stabilizers include deoxycholic acid, polyvinyl alcohol (PVA), Polyvinylpyrrolidone (PVP), and polyethylene glycol (PEG). Stabilizers and their use in the production of nanoparticles are well known to one of ordinary skill in the art. In some embodiments, physico-chemical characterization of nanoparticles is well known to one of ordinary skill in the art. For example, in some embodiments, the heterogeneity of sizes (molecular weight) of the nanoparticles can be measured by determining the dispersity of the nanoparticles, which can be expressed in terms of the polydispersity index (PDI).

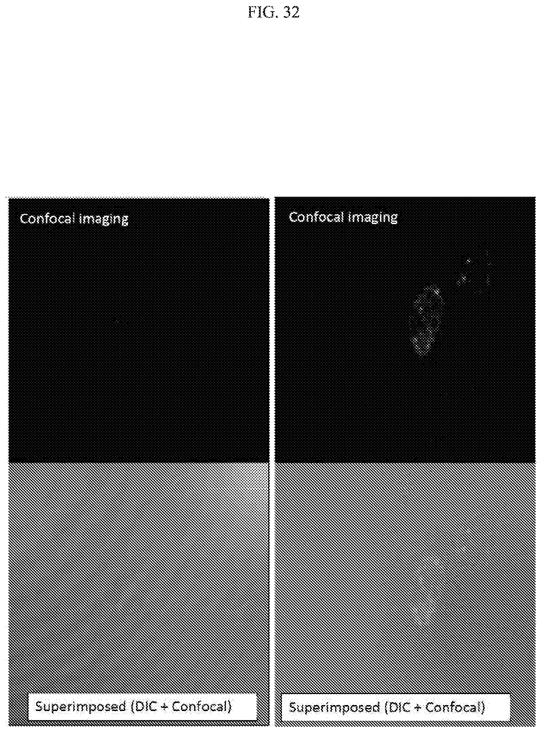

In some embodiments, the nanoparticles are conjugate to one or more small molecules (e.g. folic acid). In some embodiments, the one or more small molecules allow for the nanoparticles to be specifically taken up by target cells (e.g., brain cancer cells). Without being bound by any theory, enhanced expression of folic acid receptor is commonly found on cancer cells. Therefore, conjugating nanoparticles to folic acid can selectively enhance the uptake of the nanoparticles by cancer cells (e.g., FIG. 32). One of ordinary skill in the art can select a conjugate for modifying a nanoparticle based on the cancer(s) to be targeted. For example, in some embodiment, two or more conjugates can be used to modify a nanoparticle to specifically target the nanoparticle to more than one type of cancer.

According to the disclosure, additional treatment options can be combined with the one or more combinations disclosed herein. For example, radiation therapy can be provided and/or radiosurgery can be performed. Non-limiting examples include whole brain radiation therapy, Gamma knife, and Cyberknife. Thus, for example, in some embodiments, subjects with recurrent and/or refractory glioblastoma can be treated with a combination of nano-Quercetin (e.g., nanospheres with Quercetin) administered intravenously and Temodar administered orally along with Leading Edge^{®} Gamma Knife Technique radiation therapy.

### Additional embodiments

In some embodiments, the combinations in the compositions, and the compositions for the kits, uses and/or methods described herein comprise, consist of, or consist essentially of, Quercetin + Temodar, wherein Quercetin is administered intravenously, wherein the concentration of Quercetin in a solution for intravenous administration is about 5 mg/ml to about 500 mg/ml, and wherein Temodar is administered intravenously, wherein the Temodar is in a solution for intravenous administration.

In some embodiments, the combinations in the compositions, and the compositions for the kits, uses and/or methods described herein comprise, consist of, or consist essentially of, Quercetin + Temodar, wherein Quercetin is administered intravenously, wherein the dose of Quercetin in a solution for intravenous administration is about 0.05 g to about 10 g, and wherein Temodar is administered intravenously, wherein the Temodar is in a solution for intravenous administration.

In some embodiments, the combinations in the compositions, and the compositions for the kits, uses and/or methods described herein comprise, consist of, or consist essentially of, Quercetin + Temodar, wherein Quercetin is administered orally, wherein the amount of Quercetin in a composition for oral administration is about 100 mg to about 50 g, and wherein Temodar is administered orally, wherein the Temodar is in a composition for oral administration.

In some embodiments, the combinations in the compositions, and the compositions for the kits, uses and/or methods described herein comprise, consist of, or consist essentially of, Quercetin + Temodar, wherein Quercetin is administered in a liposomal formulation, wherein the dose of Quercetin for administration in a liposomal formulation is about 25 mg a day to about 100 mg a day, and wherein Temodar is either administered intravenously, wherein the Temodar is in a solution for intravenous administration, or wherein Temodar is administered orally, wherein the Temodar is in a composition for oral administration.

The formulations of compositions and compositions for the kits for the use as described herein include the combinations provided in Table 0.3 below.

**Table 0.3 - Combinations for formulations of compositions and compositions for the kits.**

| Combination | Temodar (Oral) | Temodar (IV) | Quercetin | | | | SPB (for reference) | | | EGCG (for reference) | | |
|---|---|---|---|---|---|---|---|---|---|---|---|---|
| | | | IV; about 5 mg/ml to about 500 mg/ml | IV; about 0.05 g to about 10g | Oral; about 100 mg to about 50 g | Liposomal; about 25 mg a day to about 100 mg a day | IV; about 20 mg/ml to about 2000 mg/ml | IV; about 0.5 g to about 100 g | Oral; about 0.1 g to about 50 g | IV: about 5 mg/ml to about 100 mg/ml | IV; about 0.01 g to about 15 g | Oral; about 0.1 g to about 3 g |
| 1. | X | | | | X | | | | | | | |
| 2. | X | | | | | X | | | | | | |
| 3. | X | | | | | | | | X | | | |
| 4. | X | | | | | | | | | | | X |
| 5. | X | | | | X | | | | X | | | |
| 6. | X | | | | X | | | | | | | X |
| 7. | X | | | | | | | | X | | | X |
| 8. | X | | | | X | | | | X | | | X |
| 9. | X | | | | | X | | | X | | | |
| 10. | X | | | | | X | | | | | | X |
| 11. | X | | | | | X | | | X | | | X |
| 12. | X | | X | | | | | | | | | |
| 13. | X | | | X | | | | | | | | |
| 14. | X | | | | | X | | | | | | |
| 15. | X | | | | | | X | | | | | |
| 16. | X | | | | | | | X | | | | |
| 17. | X | | | | | | | | | X | | |
| 18. | X | | | | | | | | | | X | |
| 19. | X | | X | | | | X | | | | | |
| 20. | X | | X | | | | | | | X | | |
| 21. | X | | | | | | X | | | X | | |
| 22. | X | | X | | | | | X | | | | |
| 23. | X | | X | | | | | | | | X | |
| 24. | X | | | | | | | X | | | X | |
| 25. | X | | | X | | | X | | | | | |
| 26. | X | | | X | | | | | | X | | |
| 27. | X | | | X | | | | X | | | | |
| 28. | X | | | X | | | | | | | X | |
| 29. | X | | | | | X | X | | | | | |
| 30. | X | | | | | X | | | | X | | |
| 31. | X | | | | | X | | X | | | | |
| 32. | X | | | | | X | | | | | X | |
| 33. | X | | X | | | | X | | | X | | |
| 34. | X | | X | | | | X | | | | X | |
| 35. | X | | X | | | | | X | | X | | |
| 36. | X | | X | | | | | X | | | X | |
| 37. | X | | | X | | | X | | | X | | |
| 38. | X | | | X | | | X | | | | X | |
| 39. | X | | | X | | | | X | | X | | |
| 40. | X | | | X | | | | X | | | X | |
| 41. | | X | | | X | | | | | | | |
| 42. | | X | | | | X | | | | | | |
| 43. | | X | | | | | | | X | | | |
| 44. | | X | | | | | | | | | | X |
| 45. | | X | | | X | | | | X | | | |
| 46. | | X | | | X | | | | | | | X |
| 47. | | X | | | | | | | X | | | X |
| 48. | | X | | | X | | | | X | | | X |
| 49. | | X | | | | X | | | X | | | |
| 50. | | X | | | | X | | | | | | X |
| 51. | | X | | | | X | | | X | | | X |
| 52. | | X | X | | | | | | | | | |
| 53. | | X | | X | | | | | | | | |
| 54. | | X | | | | X | | | | | | |
| 55. | | X | | | | | X | | | | | |
| 56. | | X | | | | | | X | | | | |
| 57. | | X | | | | | | | | X | | |
| 58. | | X | | | | | | | | | X | |
| 59. | | X | X | | | | X | | | | | |
| 60. | | X | X | | | | | | | X | | |
| 61. | | X | | | | | X | | | X | | |
| 62. | | X | X | | | | | X | | | | |
| 63. | | X | X | | | | | | | | X | |
| 64. | | X | | | | | | X | | | X | |
| 65. | | X | | X | | | X | | | | | |
| 66. | | X | | X | | | | | | X | | |
| 67. | | X | | X | | | | X | | | | |
| 68. | | X | | X | | | | | | | X | |
| 69. | | X | | | | X | X | | | | | |
| 70. | | X | | | | X | | | | X | | |
| 71. | | X | | | | X | | X | | | | |
| 72. | | X | | | | X | | | | | X | |
| 73. | | X | X | | | | X | | | X | | |
| 74. | | X | X | | | | X | | | | X | |
| 75. | | X | X | | | | | X | | X | | |
| 76. | | X | X | | | | | X | | | X | |
| 77. | | X | | X | | | X | | | X | | |
| 78. | | X | | X | | | X | | | | X | |
| 79. | | X | | X | | | | X | | X | | |
| 80. | | X | | X | | | | X | | | X | |

In Table 0.3, "X" indicates the components of the combinations for formulations of compositions and compositions for the kits, uses and/or methods described herein. Intravenous dosing of Temodar can be, but is not limited to, 40 mg/kg to 50 mg/kg in divided doses over a period of 2 to 5 days, or 10 mg/kg to 15 mg/kg every 7 to 10 days, or 3 mg/kg to 5 mg/kg twice weekly. Oral dosing Temodar can be, but is not limited to, 1 mg per kg per day to 5 mg per kg per day, or 1 mg/kg/day to 5 mg/kg/day, or 2 mg/kg/day for 8 to 12 weeks (maximum cumulative dose 168 mg per kg). In some embodiments, including those in Table 0.3, in a combination comprising Quercetin and SPB, the ratio of the concentration and/or dosing of Quercetin and SPB ranges from about 1:2 to about 1:30. In some embodiments, including those in Table 0.3, in a combination comprising Quercetin and SPB, the ratio of the concentration and/or dosing of Quercetin and SPB is about 1:10. In some embodiments, including those in Table 0.3, in a combination comprising Quercetin and SPB, the ratio of the concentration and/or dosing of Quercetin and SPB is about 1:1.25, 1:1.5, 1:1.75, 1:2, 1:4, 1:6, 1:8, 1:10, 1:12, 1:14, 1:16, 1:18, 1:20, 1:22, 1:24, 1:26, 1:28, 1:30, 1:32, 1:34, 1:36, 1:38, 1:40, or a ratio within a range defined by any two of the aforementioned ratios.

In some embodiments, including but not limited to those in Table 0.3, the dose of Quercetin ranges from about 0.5 g to about 2 g. In some embodiments, the dose of quercetin is about 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5 or 10 g, or within a range defined by any two of the aforementioned values.

### Examples

The following Examples illustrate the invention. The concentrations and doses of the anti-cancer agents and modulators disclosed in the following Examples are non-limiting. Other acceptable concentrations, ranges of concentrations, doses or ranges of doses are also contemplated.

### Example 1 - Synthesis and characterization of nanoparticles

Several nanoparticles (NPs) were synthetized and characterized. PLGA-PEG-NPs, FA-PLGA-PEG-NPs, DSPE-PEG-NPs and FA-DSPE-PEG-NPs encapsulating quercetin were synthesized. The corresponding void nanoparticles (without encapsulating quercetin) were synthesized as controls. During the process of nanoparticles synthesis, various parameters such as different types of stabilizers, amount of stabilizer, amount of drug (quercetin), etc. were tested.

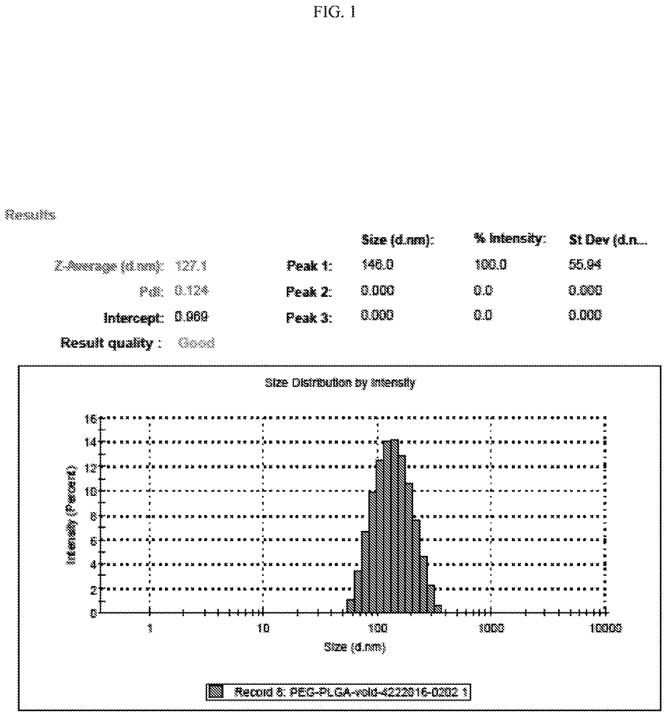

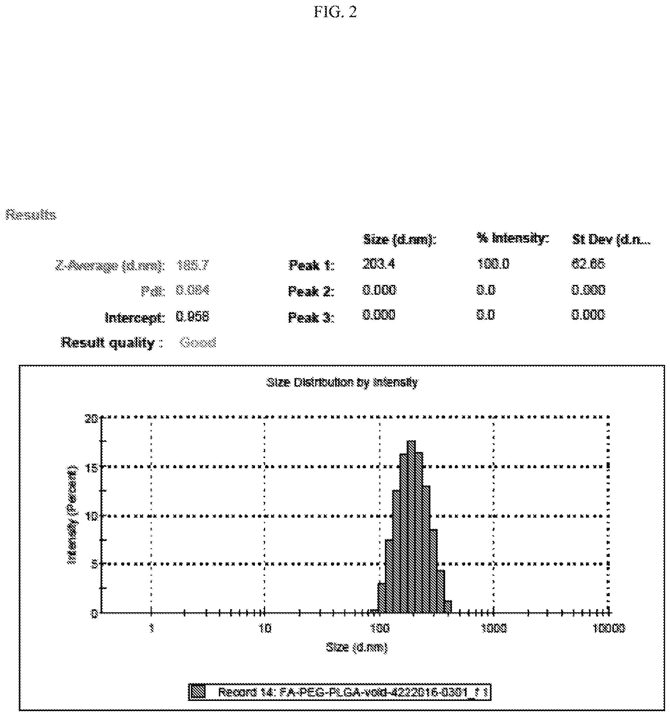

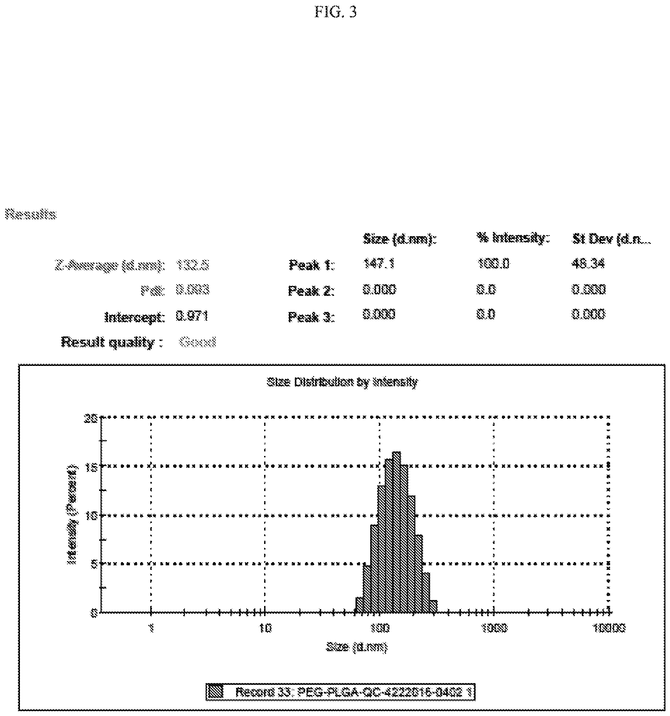

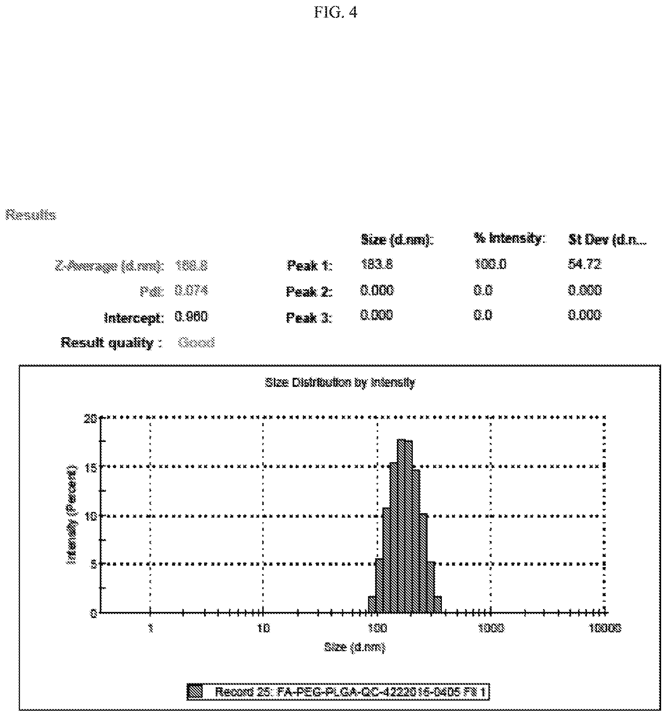

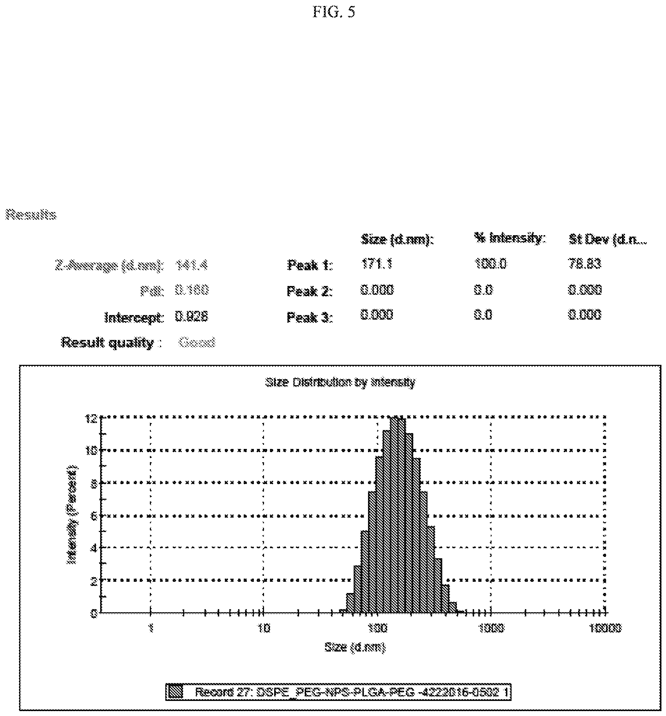

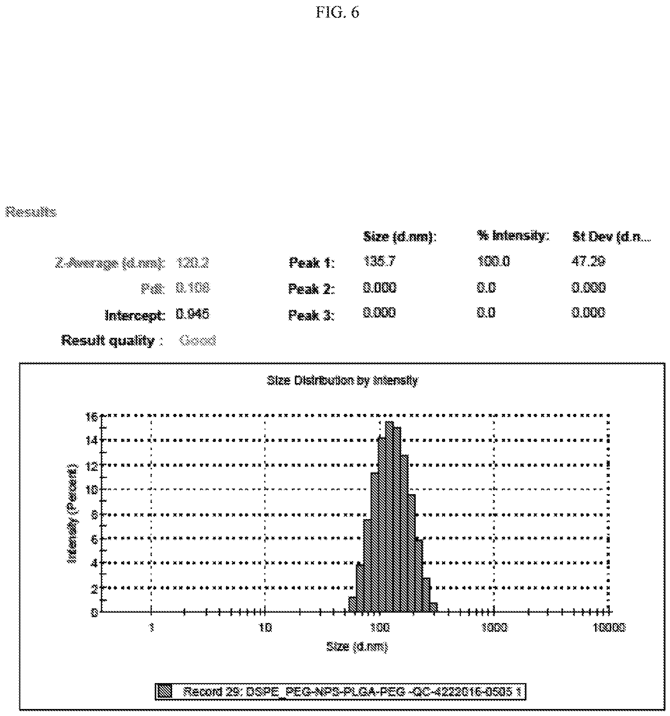

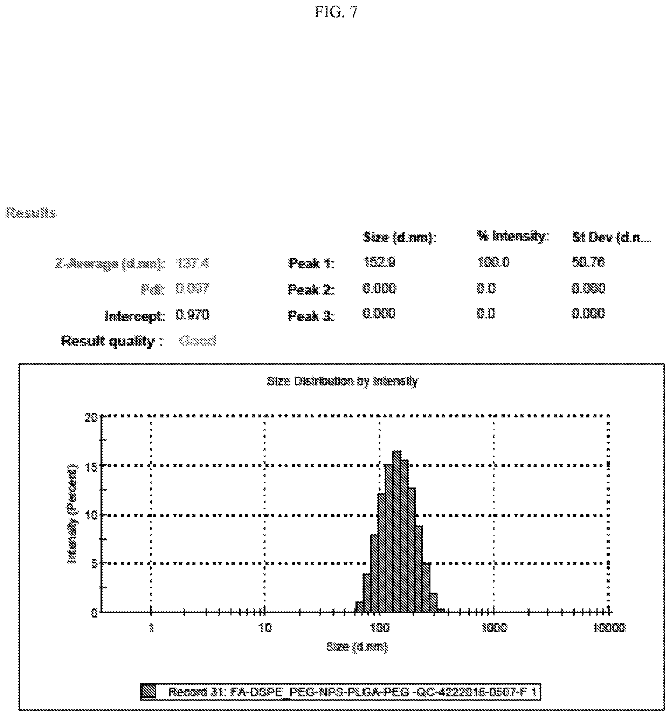

While PLGA-PEG and DSPE-PEG are commercially available, PLGA-PEG conjugated to folic acid was synthesized using carbodiimide chemistry. The nanoparticles were characterized using Dynamic Light Scattering (DLS), UV-Vis-spectrophotometer. FIG. 1 - FIG. 7 show graphs showing size distribution of the different nanoparticles.

### Example 2 - Example 5

As described in Example 2 - Example 5, before choosing the nanoformulation for in vitro and/or in vivo studies, different parameters that determine the size, amount of encapsulating drug (quercetin), stability, etc. of nanoparticles were explored. During this process, nanoparticles of different sizes were synthesized, for example, ranging from about 100 nm to about 250 nm in diameter. Different type of stabilizer like Polyvinyl alcohol (PVA), deoxycholic acid were used to explore the synthesis of the nanoformulations.

### Example 2

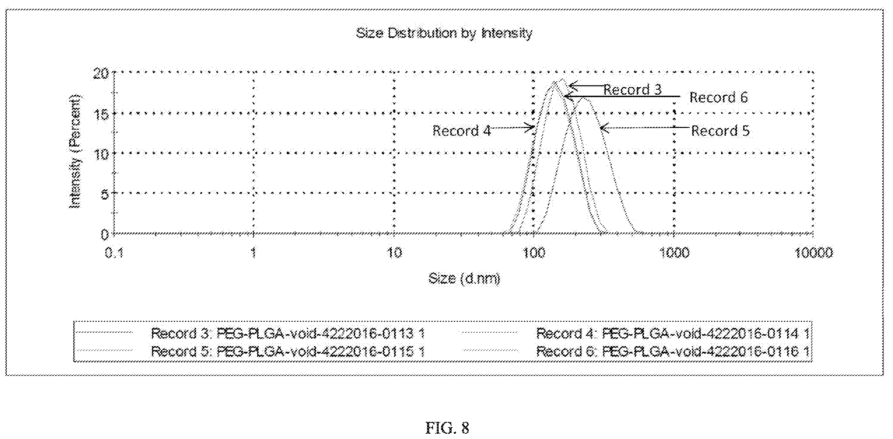

Data related to synthesis of different batches of Void-PLGA-PEG nanoparticles using polyvinyl alcohol as a stabilizer are provided (FIG. 8; TABLE 2).

**TABLE 2**

| **Record** | **Sample Name** | **Size (Z avg diameter in nm)** | **PDI** |
|---|---|---|---|
| 3 | PEG-PLGA-void-4222016-0013 | 150.9 | 0.69 |
| 4 | PEG-PLGA-void-4222016-0014 | 133.3 | 0.073 |
| 5 | PEG-PLGA-void-4222016-0015 | 220.2 | 0.152 |
| 6 | PEG-PLGA-void-4222016-0016 | 135.8 | 0.076 |

### Example 3

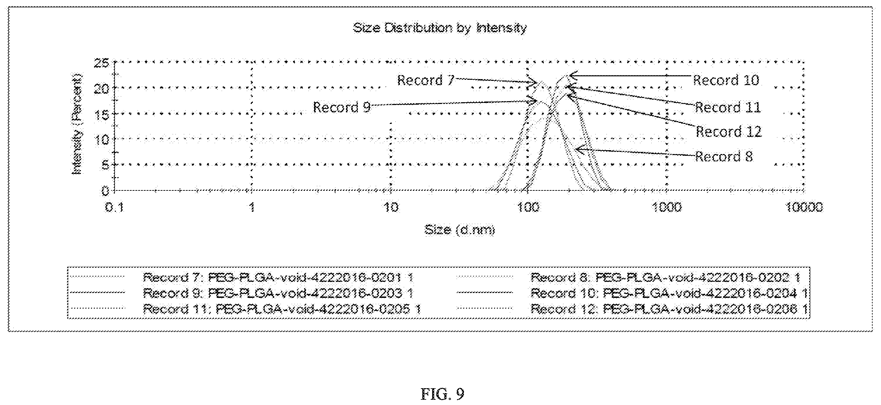

Data related to synthesis of different batches of Void-PLGA-PEG nanoparticles using deoxycholic acid as a stabilizer. Various amount of PLGA-PEG and deoxycholic acid was used to synthesized the nanoparticles are provided (FIG. 9; TABLE 3). Record 8 was selected to be tested further in *in vitro*/*in vivo* studies.

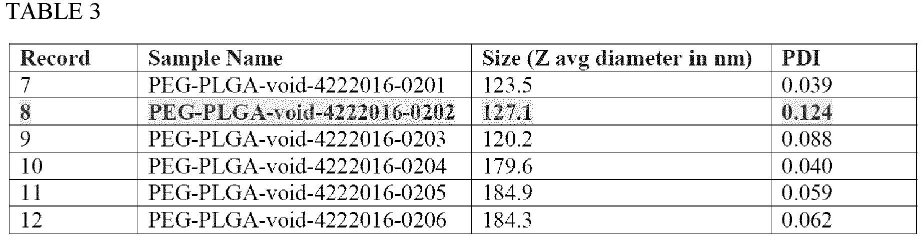

### Example 4 - Exploration of amount of Quercetin in the nanofomulations

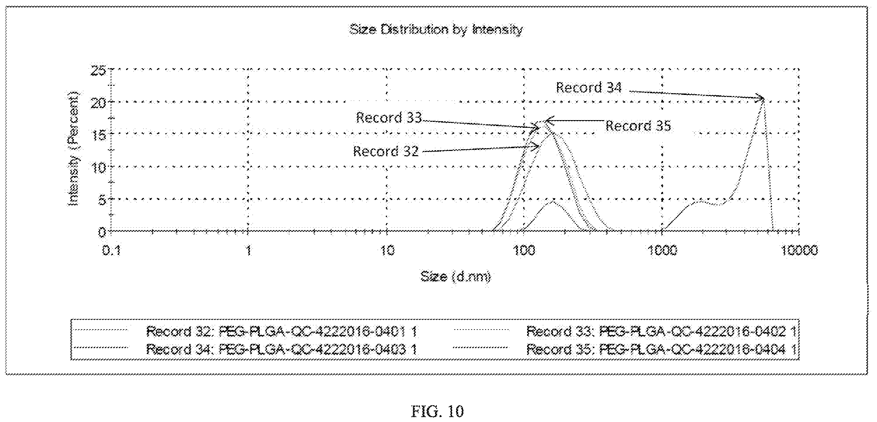

Data related to synthesis and exploration of different batches of PLGA-PEG nanoparticles encapsulating quercetin are provided. Deoxycholic acid was used as a stabilizer. Different ratios quercetin:PLGA-PEG were used to explore the synthesize process of the nanoparticles (FIG. 10; TABLE 4). Record 33 was selected to be tested further in *in vitrolin vivo* studies.

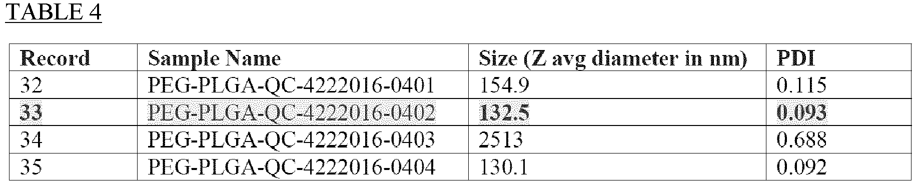

### Example 5 - DSPE-PEG-NPs synthesis and characterization

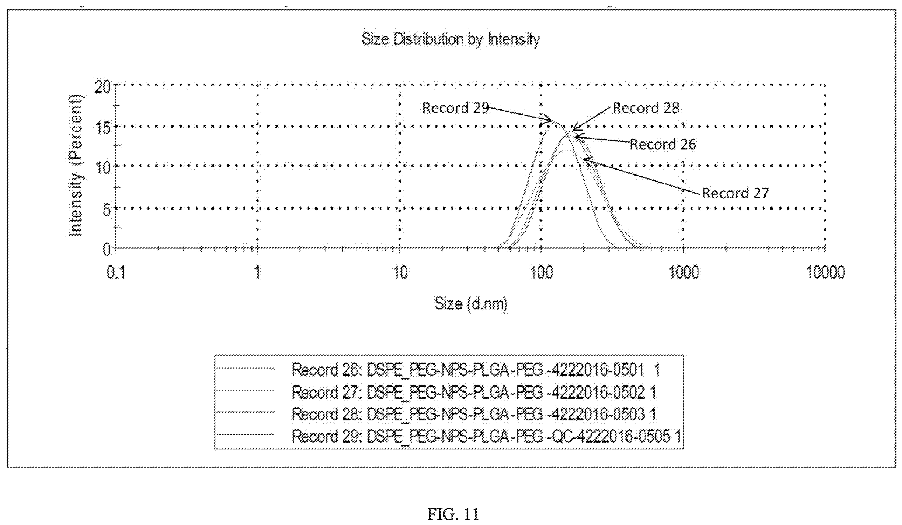

Similar to Example 4, the method for the synthesis of different batches of DSPE-PEG-NPs was optimized (FIG. 11; TABLE 5). Record 29 was selected to be tested further in *in vitro*/*in vivo* studies.

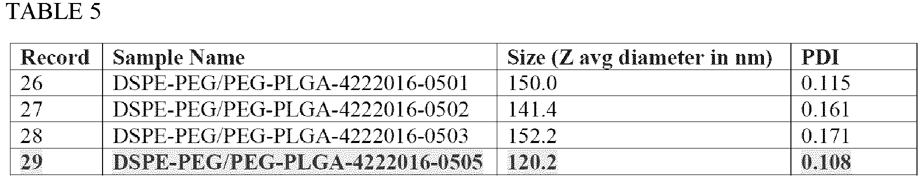

### Example 6 - Confocal imaging of the nanoparticles in cancer cells

PLGA-PEG-NPS, DSPE-PEG-NPS, FA-PEG-NPS and FA- DSPE-PEG-NPS particles labeled with a dye (Cy5) were synthesized. These nanoparticles were used to examine the preferential uptake of the nanoparticles with or without folic acid in a cancer cell line that overexpress the folic acid receptor.

FIG. 32 shows confocal imaging data showing the uptake PLGA-PEG nanoparticles encapsulating Quercetin in cancer cells overexpressing the folic acid receptor. FIG. 32, top left shows confocal image of uptake of PLGA-PEG-QC-NPs labeled with Cy5 dye without folic acid conjugation, and FIG. 32, bottom left shows superimposed confocal and DIC images of uptake of PLGA-PEG-QC-NPs labeled with Cy5 dye without folic acid conjugation. FIG. 32, top right shows confocal image of uptake of PLGA-PEG-QC-NPs labeled with Cy5 dye with folic acid conjugation, and FIG. 32, bottom right shows superimpose confocal and DIC images of uptake of PLGA-PEG-QC-NPs labeled with Cy5 dye with folic acid conjugation.

Preferential uptake by cancer cells of PLGA-PEG-QC nanoparticles conjugated to folic acid was observed (FIG. 32; top right and bottom right) compared to non-conjugated nanoparticle (i.e., not conjugated to folic acid) (FIG. 32; top and bottom left).

### Example 7 - Confocal imaging of the nanoparticles in OVACAR3 cells

PLGA-PEG-NPS, DSPE-PEG-NPS, FA-PEG-NPS and FA- DSPE-PEG-NPS particles labeled with a dye (Cy5) are synthesized. These nanoparticles are used to examine the preferential uptake of the nanoparticles with or without folic acid in an ovarian cancer cell line (OVACAR3) that overexpress the folic acid receptor.

Confocal image data are collected of the uptake of PLGA-PEG-QC-NPs labeled with Cy5 dye without folic acid conjugation. Superimposed confocal and DIC images of uptake of PLGA-PEG-QC-NPs labeled with Cy5 dye without folic acid conjugation are also collected. Confocal image data are collected of uptake of PLGA-PEG-QC-NPs labeled with Cy5 dye with folic acid conjugation. Superimposed confocal and DIC images of uptake of PLGA-PEG-QC-NPs labeled with Cy5 dye with folic acid conjugation are also collected.

Preferential uptake by cancer cells of PLGA-PEG-QC of nanoparticles conjugated to folic acid is observed compared to non-conjugated nanoparticle (i.e., not conjugated to folic acid).

### Example 8 - Xenograft study using a chemo-resistant cancer cell line

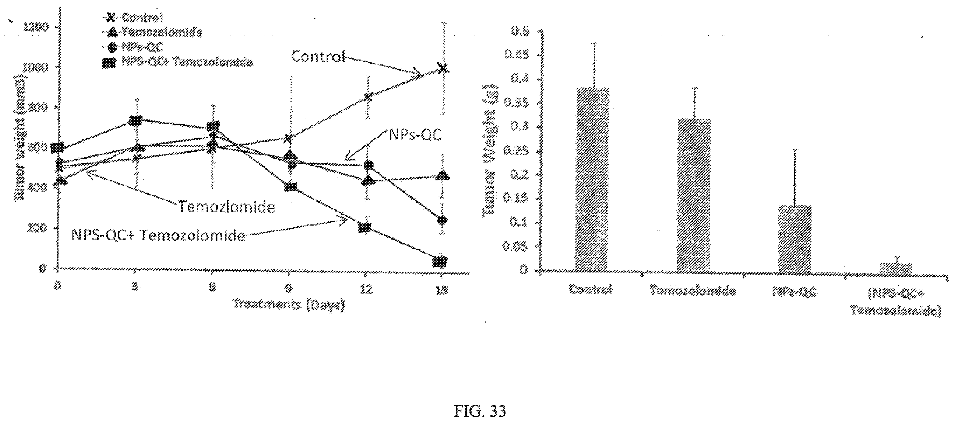

Xenograft experiments were performed using a chemo-resistant cancer cell line to determine the effect of nanoformulations on tumor growth viability (FIG. 33).

A glioma cell line (U-87 MG cells) that is resistant to/shows suboptimal response to Temozolomide was used. Immunodeficient female homozygous NCr (lacking a thymus) nude mice were used for this experiment. 5-6 weeks old mice, weighing around 20 g was used for these experiments. Mice were provided with *ad libitum* access to water and food. Cultured U-87 MG cells were harvested and implanted subcutaneously (s.c.) in both flank (5 x 10⁶ cells in 100 µl volumes containing 50% Matrigel). Tumors were allowed to grow for 5 days (to an initial volume of 500-600 mm³) before administration of a treatment. Immediately before initiation of treatment at 6 days after implantation, animals were divided into control (PBS) and treatment groups containing similar distributions of tumor volumes (calipers measurement). The treatment groups were administered Temozolomide (temodar), nanoformulations with modulator (PLGA-PEG-QC-NPs), or Temozolomide + nanoformulations with modulator (Temozolomide+ PLGA-PEG-QC-NPs). The tumor volume was measured (calipers measurements) twice in a week. After humane sacrifice of animals at 20 days (after inoculation), tumors were weighed.

Data are shown in FIG. 33. The chemo-resistant cell line continued to grow in the control sample despite presence of chemotherapy. However, a decrease in U87MG xenograft volume and tumor weight was induced by daily s.c. administration for 10 days of Temozolomide, PLGA-PEG-QC-NPs, and Temozolomide + PLGA-PEG-QC-NPs.

Similar experiments were performed with an ovarian cancer cell line resistant to Doxil (Doxorubicin). Results obtained with the ovarian cancer cell line were similar to those obtained with U-87 MG cells.

### Example 9 - Accumulation of the nano formulations in the animal with implanted tumor

Data related to studies on accumulation of the nano formulations in the animal with implanted tumor are provided.

Animals - Immunodeficient, female athymic mice aged 5-6 weeks and weighing between 18 and 20 g were purchased from Taconic Biosciences (Albany, NY). All animal studies were conducted at the animal facility of the Veteran Affairs Medical Center, Albany, NY in accordance with and approved by institutional guidelines for humane animal treatment and according to the current guidelines. Mice were maintained under specific pathogen-free conditions and housed under controlled conditions of temperature (20-24°C) and humidity (60-70%) and 12 h light/dark cycle with ad libitum access to water and food. Mice were allowed to acclimatize for 5 d prior to the start of study.

*Treatment* - OVCAR 3 Cancer cells (1 x 10⁶) were implanted subcutaneously in right flank of each animal. In the first set of experiments, control, free Quercetin (3 mg/kg), NPS-quercetin(3 mg/kg), and FA-NPs-quercetin (3 mg/kg) were injected subcutaneously to the mice everyday (for 14 days). The control was PBS, after humane sacrifice of animals at 14 days, tumors were harvested and amount of QC was measured.

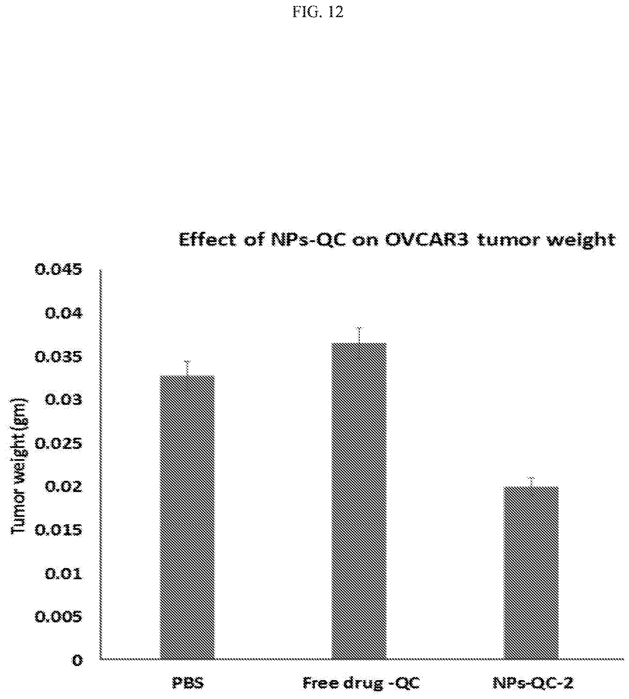

FIG. 12 shows the effect of Quercetin and nano-formulated quercetin on tumor weight. Though Free QC and NPS-QC does not have any adverse toxic effect on the health of tumor bearing mice, however, in case FA-NPS-QC there was black patch observed in the injection site and tumor (after 24 hrs. of injection).

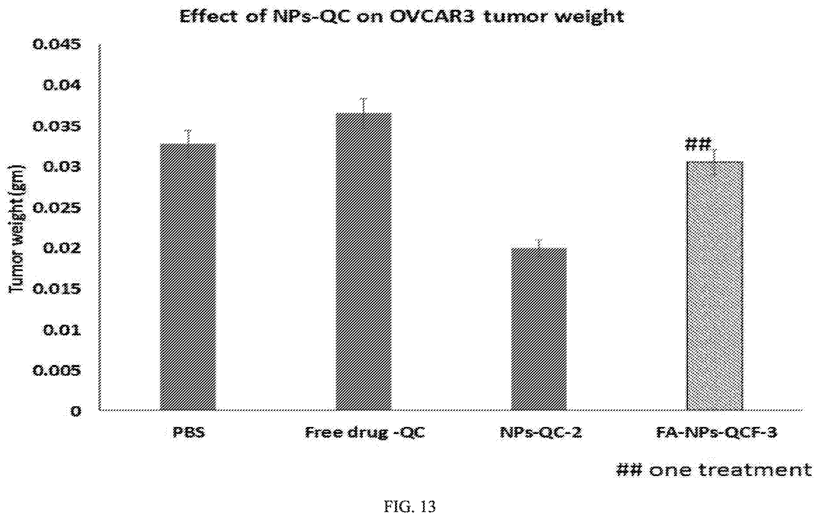

FIG. 13 shows effect of Quercetin and nano-formulated quercetin on tumor weight. Because administration of FA-NPS-QC resulted in a black patch at the injection site and tumor (after 24 hrs. of injection), the mice were administered a only one single dose of FA-QC-NPS.

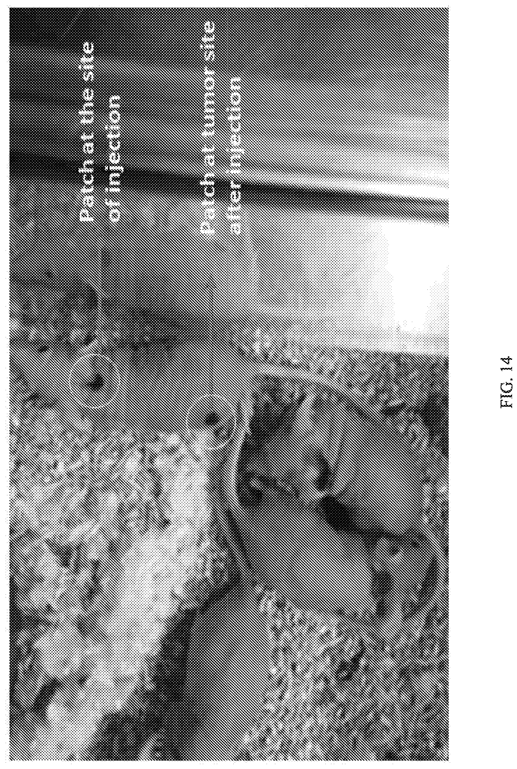

FIG. 14 shows side effects of the FA-NPS-QC on tumor bearing mice. Though this black patch was observed in 24 hours of injection, however, there was other adverse toxic effect (like weight loss, behavioral change) were not observed.

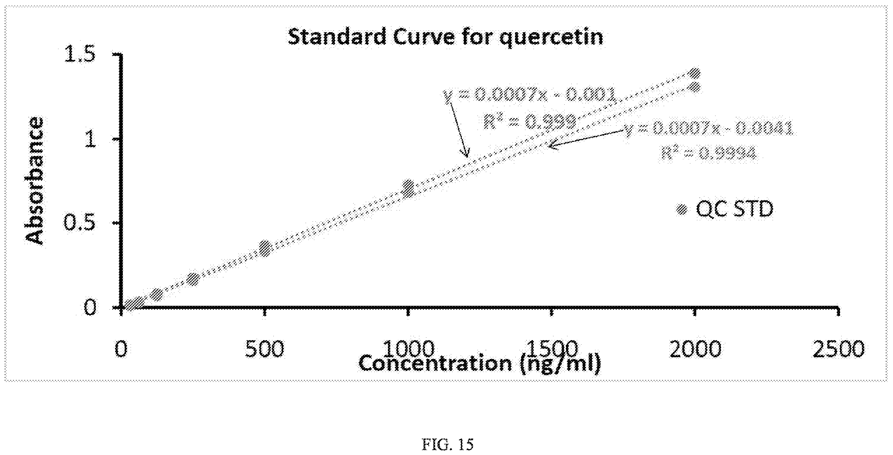

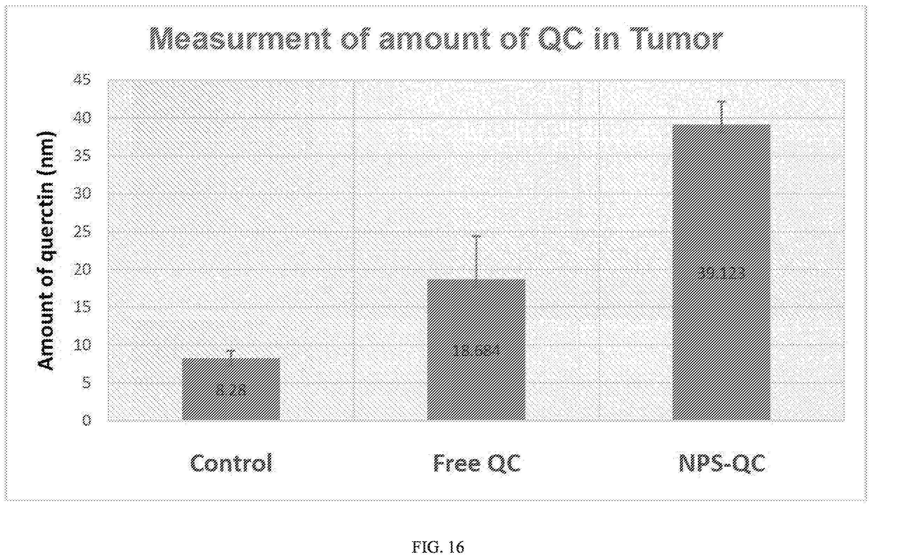

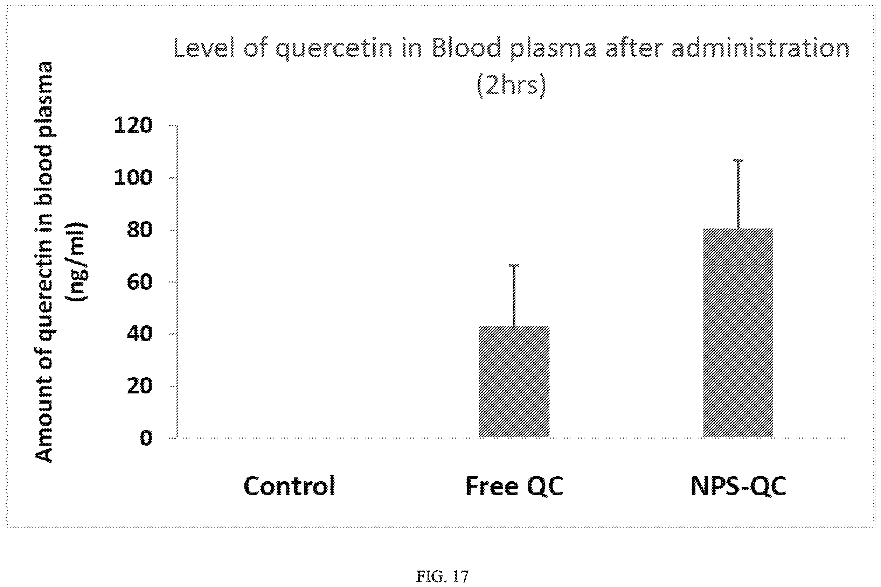

FIG. 15 shows standard curve of Quercetin (with and without spiking in the blood). For the measurement of QC levels in blood/plasma, tumor and other major organs of the treated mice, a method of measurement of QC was developed. This method was validated by spiking known amount of QC in whole blood and extracting the QC using acetonitrile and methanol solution. The method of extraction from blood/plasma, tumor and other organs were developed and it was observed that % of extraction of QC was more than 90%. FIG. 16 shows measurement of amount of quercetin in the tumor after the termination of the study. FIG. 17 shows measurement of quercetin in blood plasma after 2 hrs. of injection (subcutaneous).

### Example 10 - Treatment of Temodar-resistant neoplasm with a combination of Temodar and at least one modulator

One or more patients are identified who have one or more cancers selected from astrocytomas, pilocytic astrocytoma, diffuse astrocytoma, anaplastic astrocytoma, and brain stem gliomas, glioblastomas, glioblastomas multiforme, meningioma, gliomas, ependymomas, oligodendrogliomas, and mixed gliomas, brain tumors, pituitary tumors, craniopharyngiomas, germ cell tumors, pineal region tumors, medulloblastomas, and primary CNS lymphomas.

The one or more patients are administered a formulation comprising, consisting of, or consisting essentially of, Temodar only. Temodar is given at a dose of about 10 mg/m² to about 1000 mg/m².

On the day of the next administration and prior to the administration, as assessment of the outcome of treatment until that point is conducted and compared to all previous assessments.

Assessment of the patients after an initial period of treatment in terms of parameters such as cancer progression, growth and size, indicates that the cancers have not progressed, slowed in growth, stopped growing/progressing, reduced in size, and/or eliminated. However, following the initial response period, patients show a decline in response to Temodar. The cancers eventually become resistant to Temodar. The cancers resume progressing and growing in size.

Patients are then administered a combination comprising, consisting of, or consisting essentially of, a Temodar and at least one modulator selected from quercetin, SPB and EGCG as disclosed herein. Temodar is given at a dose of about 10 mg/m² to about 1000 mg/m². The at least one modulator is given at a dose as disclosed herein. An assessment of the outcome of treatment is periodically made.

After a minimum period of treatment (e.g., one day), the patient shows improvement. Assessments of the cancer in terms of parameters such as its progression, growth and/or size, indicates that the cancer has not progressed, slowed in growth, stopped growing/progressing, remained the same or reduced in size, and/or been eliminated. Additional assessment shows that after therapy, patients have improved in one or more measures selected from the group of: Natural Killer cell activity, increased WBC count, decreased LDH activity, decreased tumor markers, shrinkage of tumor in radiographic studies, decrease in CRP (correlation with improved survival), improved IgF-l. In some cases, the patients do not show a decline in response to the combination comprising, consisting of, or consisting essentially of, a Temodar plus at least one modulator selected from quercetin, SPB and EGCG as disclosed herein during the course of the treatment, and the cancers are eventually eliminated without relapse.

### Example 11 - Treatment of neoplasm with Temodar and at least one modulator

One or more patients are identified who have one or more cancers selected from astrocytomas, pilocytic astrocytoma, diffuse astrocytoma, anaplastic astrocytoma, and brain stem gliomas, glioblastomas, glioblastomas multiforme, meningioma, gliomas, ependymomas, oligodendrogliomas, and mixed gliomas, brain tumors, pituitary tumors, craniopharyngiomas, germ cell tumors, pineal region tumors, medulloblastomas, and primary CNS lymphomas.

Patients are then administered a combination of Temodar and at least one modulator selected from quercetin, SPB and EGCG as disclosed herein. Temodar is given at a dose of about 10 mg/m² to about 1000 mg/m². The at least one modulator is given at a dose as disclosed herein. An assessment of the outcome of treatment is periodically made.

After a minimum period of treatment (e.g., one day) at a minimum dose required to prevent and/or treat the neoplasm, the patient shows improvement. Assessments of the cancer in terms of parameters such as its progression, growth and/or size, indicates that the cancer has not progressed, slowed in growth, stopped growing/progressing, remained the same or reduced in size, and/or been eliminated. Additional assessment shows that after therapy, patients have improved in one or more measures selected from the group of Natural Killer cell activity, increased WBC count, decreased LDH activity, decreased tumor markers, shrinkage of tumor in radiographic studies, decrease in CRP (correlation with improved survival), improved IgF-l. In some cases, the patients do not show a decline in response to the combination of Temodar plus at least one modulator selected from quercetin, SPB and EGCG as disclosed herein during the course of the treatment, and the cancers are eventually eliminated without relapse.

### Example 12 - Treatment of Temodar-resistant neoplasm with a combination of Temodar and at least one modulator in a nanoparticle

One or more patients are identified who have one or more types of brain cancers selected from astrocytomas, pilocytic astrocytoma, diffuse astrocytoma, anaplastic astrocytoma, and brain stem gliomas, glioblastomas, glioblastomas multiforme, meningioma, gliomas, ependymomas, oligodendrogliomas, and mixed gliomas, brain tumors, pituitary tumors, craniopharyngiomas, germ cell tumors, pineal region tumors, medulloblastomas, and primary CNS lymphomas.

The one or more patients are administered a formulation comprising, consisting of, or consisting essentially of, Temodar only. Temodar is given at a dose of about 10 mg/m² to about 1000 mg/m².

On the day of the next administration and prior to the administration, as assessment of the outcome of treatment until that point is conducted and compared to all previous assessments.

Assessment of the patients after an initial period of treatment in terms of parameters such as cancer progression, growth and size, indicates that the cancers have not progressed, slowed in growth, stopped growing/progressing, reduced in size, and/or eliminated. However, following the initial response period, patients show a decline in response to Temodar. The cancers eventually become resistant to Temodar. The cancers resume progressing and growing in size.

Patients are then administered a nanoparticle formulation comprising, consisting of, or consisting essentially of, a combination of Temodar and nano-quercetin. Temodar is given at a dose of about 10 mg/m² to about 1000 mg/m². Nano-Quercetin is given at a dose of Quercetin as disclosed herein. An assessment of the outcome of treatment is periodically made.

After a minimum period of treatment (e.g., one day), the patient shows improvement. Assessments of the cancer in terms of parameters such as its progression, growth and/or size, indicates that the cancer has not progressed, slowed in growth, stopped growing/progressing, remained the same or reduced in size, and/or been eliminated. Additional assessment shows that after therapy, patients have improved in one or more measures selected from the group of: Natural Killer cell activity, increased WBC count, decreased LDH activity, decreased tumor markers, shrinkage of tumor in radiographic studies, decrease in CRP (correlation with improved survival), improved IgF-l. In some cases, the patients do not show a decline in response to the combination comprising, consisting of, or consisting essentially of, a Temodar plus quercetin during the course of the treatment, and the cancers are eventually eliminated without relapse.

### Example 13 - Treatment of neoplasm with a nanoformulation of Temodar and at least one modulator

One or more patients are identified who have one or more types of brain cancer selected from astrocytomas, pilocytic astrocytoma, diffuse astrocytoma, anaplastic astrocytoma, and brain stem gliomas, glioblastomas, glioblastomas multiforme, meningioma, gliomas, ependymomas, oligodendrogliomas, and mixed gliomas, brain tumors, pituitary tumors, craniopharyngiomas, germ cell tumors, pineal region tumors, medulloblastomas, and primary CNS lymphomas.

Patients are then administered a nanoparticle formulation comprising, consisting of, or consisting essentially of, a combination of Temodar and quercetin, in which at least one or both are present as nanoparticles. Temodar is given at a dose of about 10 mg/m² to about 1000 mg/m². Quercetin is given at a dose as disclosed herein. An assessment of the outcome of treatment is periodically made.

After a minimum period of treatment (e.g., one day), the patient shows improvement. Assessments of the cancer in terms of parameters such as its progression, growth and/or size, indicates that the cancer has not progressed, slowed in growth, stopped growing/progressing, remained the same or reduced in size, and/or been eliminated. Additional assessment shows that after therapy, patients have improved in one or more measures selected from the group of: Natural Killer cell activity, increased WBC count, decreased LDH activity, decreased tumor markers, shrinkage of tumor in radiographic studies, decrease in CRP (correlation with improved survival), improved IgF-l. In some cases, the patients do not show a decline in response to the combination of Temodar plus quercetin during the course of the treatment, and the cancers are eventually eliminated without relapse.

### Example 14 - Treatment of neoplasm with a nanoformulation of Temodar and at least one modulator

One or more patients are identified who have one or more types of brain cancer selected from astrocytomas, pilocytic astrocytoma, diffuse astrocytoma, anaplastic astrocytoma, and brain stem gliomas, glioblastomas, glioblastomas multiforme, meningioma, gliomas, ependymomas, oligodendrogliomas, and mixed gliomas, brain tumors, pituitary tumors, craniopharyngiomas, germ cell tumors, pineal region tumors, medulloblastomas, and primary CNS lymphomas.

Patients are then administered a nanoparticle formulation comprising, consisting of, or consisting essentially of, a combination of Temodar and quercetin, in which both are present as nanoparticles. Temodar is given at a dose of about 10 mg/m² to about 1000 mg/m². Quercetin is given at a dose of as disclosed herein. An assessment of the outcome of treatment is periodically made.

After a minimum period of treatment (e.g., one day) at a minimum dose required to prevent and/or treat the neoplasm, the patient shows improvement. Assessments of the cancer in terms of parameters such as its progression, growth and/or size, indicates that the cancer has not progressed, slowed in growth, stopped growing/progressing, remained the same or reduced in size, and/or been eliminated. Additional assessment shows that after therapy, patients have improved in one or more measures selected from the group of Natural Killer cell activity, increased WBC count, decreased LDH activity, decreased tumor markers, shrinkage of tumor in radiographic studies, decrease in CRP (correlation with improved survival), improved IgF-l. In some cases, the patients do not show a decline in response to the combination of Temodar plus quercetin during the course of the treatment, and the cancers are eventually eliminated without relapse.

### Example 15 - Case Study - Lisa - Treatment of metastatic melanoma with Quercetin PG-SPB, and Quercetin PG-Temodar

A 61 year old female patient presented at the clinic of Dr. Nezami for evaluation and management of refractory and metastatic melanoma. She was first diagnosed 11 years earlier, starting as a mole on her right abdomen that was biopsied and was shown to be superficial spreading melanoma. The pathology showed that the tumor had a low mitotic rate and no satellites. She refused conventional immune therapies, including interleukin 2 (IL-2) and other conventional therapies. Nine months prior to presenting to at the clinic of Dr. Nezami, she felt a mass under her right arm which was painful. It was initially treated as an abscess. She had been administered antibiotics for eight weeks with severe reactions. However, the lump never resolved. A biopsy of the lump and a lymph node 8 weeks later revealed metastatic melanoma. One month later she was treated with approved first line therapy of PD-1 inhibitor, nivolumab (6 injections), along with other alternative therapies/ nutritional support.

A CT scan four months later showed progression of disease. At this time, she was treated with combinational drugs Nivolumab and Ipilmumab (Optivo and Yervoy) based on her PDL-1 positive pathology. She had a severe adverse reaction to the medications with fevers, thyroid storm, electrolyte imbalance, and other endocrine problems. She had also exhausted other off label drugs without success (including naltrexone, curcumin, vitamin C, and other immune therapies). She reported that the tumor under her axilla had been steadily growing, causing pain and discomfort.

Upon her arrival at the clinic of Dr. Nezami, her disease was staged with whole body PET scan. The scan showed progressive disease (after Yervoy^{®} and Opdivo^{®} therapy) with increased chest wall mass metabolic activity and presence of cervical, periportal and peripancreatic lymph nodes. At the time, options of care were conventionally limited as she had failed immune therapies and had significant toxicity.

Laboratory tests also confirmed increased S100 calcium-binding protein B (S100B) marker (at 278), as well as presence of circulatory tumor cells (CTC) with positive markers. S100B is a protein of the S-100 protein family, and is a molecular marker of severe traumatic brain injury. Her molecular profiling of her CTC showed a negative MGMT.

At the start of her treatment, molecular detection of of circulating tumor cells was performed as follows. In order to obtain circulating tumor cells from the patient's peripheral blood, large cells and cell-clusters as well as epithelial cells were isolated. A preparation of mononuclear cells (MNC) served as a control cell fraction. mRNA was ioslated from all fractions. Thereafter, the expression of tumor relevant genes was measured by quantitative real-time RT-PCR. A preparation of mononuclear cells (MNC) served as a control cell fraction. The expression of the telomerase gene can be increaed in most tumor types but not in normal tissue. An increased expression of telomerase gene may be indicative of the presence of tumor cells in the circulation. Overexpression of telomerase was not detected in the isoalted cells. Overexpression of C-MYC indicates an increased proliferation of the ioslated cells. An increased proliferation rate is a typical feature of tumor cells. The expression of level of C-MYC was not elevated. C-KIT is a growth factor receptor which may be overexpressed in differnet kinds of tumors. N more than 50% of early stage melanomas, expression of C-KIT has been described. Elevated expression of C-KIT was detected in the isolated cells. Tyrosinase and MART1 are specifically expressed in skin be observed in breast cancer. Detection of expression of Tyrosinase-mRNA or MART1 in blood indicated circulating melanoma cells. Expression of Tyrosinase and MART1 was not detected. Thus, in the isolated tumor cell fraction, expression of C-KIT was above threshold (>2.0) and expression of CK19 was above threshold (>0) in the very high range (>10000). This finding was indicative of the likely presence of circulating tumor cells in the analyzed blood sample.

She was immediately started on IV epigenetic therapies, consisting of Quercetin PG as the mainstay of therapy, and sodium phenyl butyrate (SPB), which she received on daily basis for 4 weeks. Her quality of life improved and she did not experience any side effects from the therapy. Her laboratory tests were repeated after 2 weeks of initiating therapy and showed decreased S100B, as well as reduction of her CTC. This tumor marker has prognostic value and is correlated with survival. This decrease was accomplished without use of chemotherapy.

Two months after presenting at the clinic of Dr. Nezami, she was started on Temodar, on 60 percent dosage (calculated based on her body surface area) at 300 mg/m² (1-5/28) day schedule in combination with intravenous epigenetic therapies consisting of Quercetin PG as mainstay of therapy.

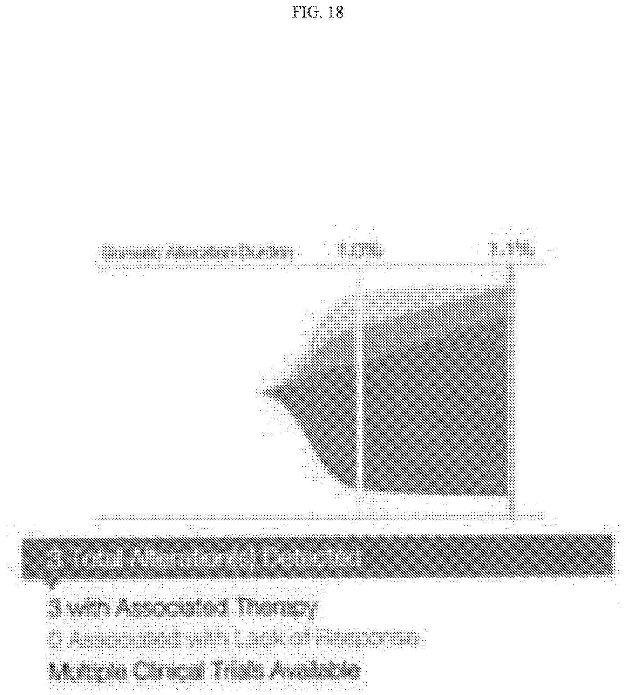

She underwent a restaging PET scan after completing two cycles of low dose Temodar and epigenetic therapies as described which showed significant positive metabolic response to the therapy with reduction of axillary mass metabolic activity ((standardized uptake values) (SUVs)) from 17.7 to 5.5. Both her para-aortic and peri-pancreatic nodes were reduced in metabolic activity and stable in size. All cervical lymph nodes were also stable. Her circulating DNA (% cfDNA - The "Somatic Alteration Burden" value in FIG. 18 refers to the maximum % cfDNA detected at each time point) improved with treatment, with reduction of mutation allele fraction (MAF) of TP53, as measured after 4 months of initiating treatment. Her S100B decreased from 278 to 179 in two months after initiation of treatment.

Further, she had her right axillary mass removed through a debulking surgery along with 66 axillary level 3 lymph nodes. She had a debulking of her 12.5 cm right axillary mass and associated 66 lymph nodes 4 months after initiation of treatment with negative cytology in all 66 nodes. The pathology confirmed that all lymph nodes were cancer free without any evidence of melanoma. About 3 weeks after initiating treatment at the clinic of Dr. Nezami, the expression level of the molecular tumor marker C-KIT had significantly decreased from 8.28 to 3.48. All other detection markers were still in the normal range. The measured values of the detection markers suggested a decrease of the tumor cell burden in the blood. Her restaging PET scan 7 months after initiation of treatment showed complete resolution of all metastatic lesions in her abdomen and pelvis as well as cervical nodes. No sign of any residual cancer was seen.

She continues to receive treatment at the clinic of Dr. Nezami with improved quality of life.

In summary, application of the disclosed combination therapy comprising, consisting of, or consisting essentially of, Temodar and Quercetin PG yielded unexpected results and showed significant effectiveness in treating metastatic melanoma. Therefore, this combination therapy is a feasible and clinically relevant option for treating metastatic melanoma. It is believed that such therapy could replace the current standard of care of metastatic melanoma.

### Example 16 - Case Study - Marcia - Treatment of glioblastoma with Quercetin PG-Temodar

This is a case study of a 70 year old female patient with history of glioblastoma. About one week after being diagnosed she underwent post MRI guided stereotactic frontal lobe craniotomy and further treatment with leading edge gamma knife radiotherapy. Temodar treatment was started immediately after diagnosis and stopped 3 months later per standard protocol. She had received 35 sessions of radiation and gamma knife at Hoag Hospital and was referred to the clinic of Dr. Nezami by her neurosurgeon for evaluation and treatments.

Upon arrival at the clinic of Dr. Nezami, she had foot drop/heavy left foot and was unable to walk. Her other neurological symptoms had recovered post craniotomy, including left hand discoordination. She also had a history of deep vein thrombosis that was treated with Eliquis around the time of her referral to the clinic of Dr. Nezami.

Upon her arrival, laboratory tests were performed which showed increased circulating DNA (% cfDNA) detected with 1.5 percent mutation allele fraction (MAF) and two alterations, neurofibromatosis 1 (NF-1) and NTRK3. She also had high LDH levels.

She was started on IV epigenetic therapies described in this application consisting of intravenous Quercetin PG as mainstay of therapy, in conjunction with Temodar as standard of care. Epigenetic therapies were received on daily basis, for ten treatments in two weeks period. Thereafter, she was re-assessed, and laboratory tests confirmed disappearance of NF-1 post therapy.

Her brain MRI was repeated about 3 months after initiation of treatment which did not show any sign of tumor progression/tumor recurrence. Her MRI again was repeated about 2 weeks later which showed improvement - the corpus callosum was no longer involved. At this time, patient had already received 17 treatments.

Her labs indicated a response to the therapies, evident by her LDH decreasing from 477 to 180, measured about 3 months and 4 months after initiation of treatment, respectively.

At the start of her treatment, molecular detection of of circulating tumor cells was performed as follows. In order to obtain circulating tumor cells from the patient's peripheral blood, large cells and cell-clusters as well as epithelial cells were isolated. A preparation of mononuclear cells (MNC) served as a control cell fraction. mRNA was ioslated from all fractions. Thereafter, the expression of tumor relevant genes was measured by quantitative real-time RT-PCR. A preparation of mononuclear cells (MNC) served as a control cell fraction. The expression of the telomerase gene can be increaed in most tumor types but not in normal tissue. An increased expression of telomerase gene may be indicative of the presence of tumor cells in the circulation. Expression of telomerase was not detected in the isoalted cells. Abnormal expression of epidermal growth factor receptor (EGFR) can be detected in many different types of cancer. Abnormal EGFR expression is therefore indicative for the presence of circulating tumor cells. EGFR expression was not detected. Overexpression of ERBB2 (HER-2/NEU) is a trait of different types of cancer and may also be observed in breast cancer. Thus, the detection of ERBB2 overexpression may be indicative of the preence of ciruclaitng tumor cells. The expression of ERBB2 was slightly elevated. C-KIT is a growth factor receptor which may be overexpressed in differnet kinds of tumors. N more than 50% of early stage melanomas, expression of C-KIT has been described. Expression of C-KIT was not detected in the isolated cells. Thus, in the isolated tumor cell fraction, expression of ERBB2 was above threshold (>2.0). This finding was indicative of the likely presence of circulating tumor cells in the analyzed blood sample.

Her circulating tumor cells (CTC) were positive for ERBB2 checked prior to the therapy (at 6.85). However, her CTC analysis 6 weeks post treatment, showed that the expression level of the molecular tumor marker ERBB2 had significantly decreased but was still above the threshold (at 2.67).

This was a very important sign of response as studies have shown that circulatory tumor cells/CTC correlate with tumor progression and response beyond imaging findings. For example, "CTCs, is superior to MRI in monitoring the treatment response in glioblastoma." *See,* https://www.ncbi.nlm.nih.gov/pmc/articles/PMC5342081/. Her MRI was repeated one month later which showed stable findings, with no sign of recurrence, no changes in satellite periventricular enhancements post-surgery. No new lesion was identified.

The patient is continuing her treatment at the clinic of Dr. Nezami, with no sign of tumor recurrence and/or progression for over 8 months. Her steroid requirement for cerebral edema is zero. Her quality of life is at the best level (ECOG of 1).

In summary, application of the disclosed combination therapy comprising, consisting of, or consisting essentially of, Temodar and Quercetin PG showed significant effectiveness in treating glioblastoma. Therefore, this combination therapy is a feasible and clinically relevant option for treating glioblastoma. It is believed that such therapy could replace the current standard of care of glioblastoma.

### Example 17 - Case Study - Maria - Treatment of brain cancer with Quercetin PG-Temodar

This study is related to a 57 year old female patient with history of breast inflammatory ductal carcinoma, ER +, PR-, Her-2 +++ status post neoadjuvant chemotherapy (Taxotere, Herceptin) administered 2 months after diagnosis and mastectomy 3 months after diagnosis. The patient received adjuvant chemotherapy for two months after mastectomy, switched to daily letrozole and herceptin every three weeks, status post recurrence 1 month later, with brain metastasis, and received radiation therapy starting 1 month later for 9 months (the last one with cyberknife). The patient further progressed with pulmonary metastasis and chest wall recurrence as determined 3 months after the last radiation therapy despite using several lines of chemotherapies, including tykerb and Xeloda combination, as well as Kadcyla (TDM-1) which was administered during the penultimate month of radiation therapy. She also received Doxil and dexamethasone for her progressed brain metastasis and was unable to walk independently. The patient then traveled from Italy for evaluation and therapy at the clinic of Dr. Nezami.

Initial evaluation revealed elevated tumor markers (CEA at 50, CA 15-3 at 362, LDH at 840, IL-8 at 43.5) as well as serum Her-2 level at 849. She was re-staged with a PET/CT, which showed significantly FDG avid lesions in her right chest wall, all thoracic, hilar, mediastinal lymph nodes, as well as left axillary nodes with SUV activity of 12, neck, subclavicular, and a large right pleural effusion. Extensive right lung densities, with right mid lung with associated very high 18F-fluorodeoxyglucose (FDG) activity (SUV of over 8). There was also an increased uptake at the right cerebellum with left mass with central photopenia, suggestive of malignancy (active metabolic tumor), occupying the left cerebellum in the brain.

She was immediately started on IV epigenetic therapies consisting of intravenous Quercetin PG as mainstay of therapy and combination of conventional therapies, in this case, Temodar, which she received on daily basis for five days a month. Her labs were repeated four days later, and the following results were obtained: Her CA 15-3 decreased from 362 to 271.6 and further to 251 and 208 about three weeks after starting treatment (on 7/17/15). Her LDH decreased from 840 to 430 and further to 393. Her CEA decreased from 50 to 47 and then 42. CA 27.29 decreased from 392 to 300. Her serum Her-2 decreased from 840 to 659. ECOG score improved from 3 to 1. She was now able to walk independently after two weeks of therapy.

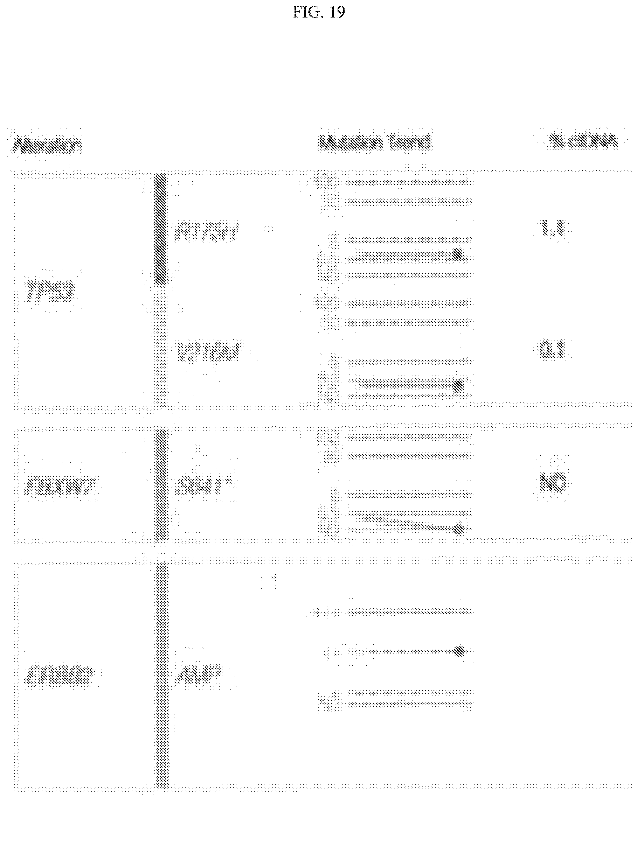

A Guardant360 Tumor Response Map (FIG. 18) illustrates the relative changes of observed in cell-free DNA (cfDNA)) at different sample submission time points. The "Somatic Alteration Burden" value (e.g., in FIG. 18) refers to the maximum % cfDNA detected at each time point. Amplifications are not plotted, and only the first and last four test dates are plotted. The percentage, or allele frequency, of altered % cfDNA circulating in blood is related to the unique tumor biology of each patient (e.g., in FIG. 19). Factors that may affect the % cfDNA of detected somatic alterations include tumor growth, turn-over, size, heterogeneity, vascularization, disease progression, and treatment. The genes covered by the Guardant360 Tumor Response Map are listed in TABLE 6.

**TABLE 6 - Genes detected by Guardant360 Tumor Response Map**

| Complete Sequencing of CoveredExons | | | | | | | | | | | |
|---|---|---|---|---|---|---|---|---|---|---|---|
| **Point Mutations (SNVs) (73 Genes)** | | | | | | | **Indels (23 Genes)** | | **Amplications (CNVs) (18 Genes)** | | **Fusions (6 Genes)** |
| AKT1 | ALK | APC | AR | ARAF | ARID1A | ATM | ATM | APC | AR | BRAF | ALK |
| BRAF | BRCA1 | BRCA2 | CCND1 | CCND2 | CCNE1 | CDH1 | ARID1A | BRCA1 | CCND1 | CCND2 | FGFR2 |
| CDK4 | CDK6 | CDKN2A | CTNNB1 | DDR2 | EGFR | ERBB2 | BRCA2 | CDH1 | CCNE1 | CDK4 | FGFR3 |
| ESR1 | EZH2 | FBXW7 | FGFR1 | FGFR2 | FGFR3 | GATA3 | CDKN2A | EGFR | CDK6 | EGFR | NTRK1 |
| GNA11 | GNAQ | GNAS | HNF1A | HRAS | IDH1 | IDH2 | ERBB2 | GATA3 | ERBB2 | FGFR1 | RET |
| JAK2 | JAK3 | KIT | KRAS | MAP2K1 | MAP2K2 | MAPK1 | KIT | MET | FGFR2 | KIT | ROS1 |
| MAPK3 | MET | MLH1 | MPL | MTOR | MYC | NF1 | MLH1 | MTOR | KRAS | MET | |
| NFE2L2 | NOTCH1 | NPM1 | NRAS | NTRK1 | NTRK3 | PDGFRA | NF1 | PDGFRA | MYC | PDGFRA | |
| PIK3CA | PTEN | PTPN11 | RAFI | RBI | RET | RHEB | PTEN | RB1 | PIK3CA | RAF1 | |
| RHOA | RIT1 | ROS1 | SMAD4 | SMO | STK11 | TERT** | SMAD4 | STK11 | | | |
| TP53 | TSC1 | VHL | | | | | TP53 | TSC1 | | | |

| | | | | | | | | | | | |
|---|---|---|---|---|---|---|---|---|---|---|---|
| **includes TERT promoter region VHL **Exons selected to maximize detection of known somatic mutations. List available upon request.* | | | | | | | | | | | |

Results of % cfDNA detected are categorized as follows: ND represents genomic alterations not detected. Genomic alterations may be present that are below the limit of detection of this test. Certain sample or variant characteristics may result in reduced analytic sensitivity, such as poor sample quality or improper collection. Genomic alterations in a tumor may be present but are not detected in circulating cell-free DNA from this blood specimen with this test. Similar to other alterations in circulating cfDNA, the amount (% cfDNA) of this variant may reflect disease progression or response to treatment. Therefore, clinical correlation is advised. As the absolute number of copies in circulation is dependent on both tumor fraction and the magnitude of the tumor amplification, amplifications are reported on a semi-quantitative scale. Positive (+) refers to amplification magnitude is in the lower 50^{th} percentile of samples with amplifications; Strongly Positive (++) refers to amplification magnitude is in the 50th to 90th percentile; Very Strongly Positive (+++) refers to amplification magnitude is in the top 10th percentile. Positive (+) refers to amplification magnitude is in the lower 50^{th} percentile of samples with amplifications; Strongly Positive (++) refers to amplification magnitude is in the 50th to 90th percentile; Very Strongly Positive (+++) refers to amplification magnitude is in the top 10th percentile.

Prior to the initiation of treatment, somatic alterations were detected in the circulating cell-free DNA isolated from this patient's blood specimen. These genomic alterations are cancer-associated somatic variants, some of which have been associated with either increased or reduced clinical response to specific treatments. Amplification was detected in the circulating cell-free DNA isolated from this patient's blood specimen for the annotated gene(s). Unlike tissue-based gene amplification tests (e.g., IHC or FISH), Guardant360 Tumor Response Map assesses the total representation of a given gene in all circulating cell-free DNA present in the patient's blood sample including material derived from the tumor and healthy tissue alike. As such, the absolute level of amplification present in the blood depends both on the tumor-derived cfDNA content and on the degree of amplification within that fraction and cannot be inferred from bulk cfDNA interrogation. For example, a positive Guardant360 Tumor Response Map test could represent a small population of cells with extremely high levels of the detected gene amplification. Alternatively, it could represent a large population of cells with low to medium levels of the detected gene amplifications. The exact correlation between amplification detected by the Guardant360 Tumor Response Map compared to IHC or FISH and how each test differentially guides patient management is an area of active investigation.

The Guardant360 Tumor Response Map confirmed the presence of circulatory DNA that were positive for mutation of oncosuppresor genes FBXW7, ERBB2, and P53 (FIG. 19).

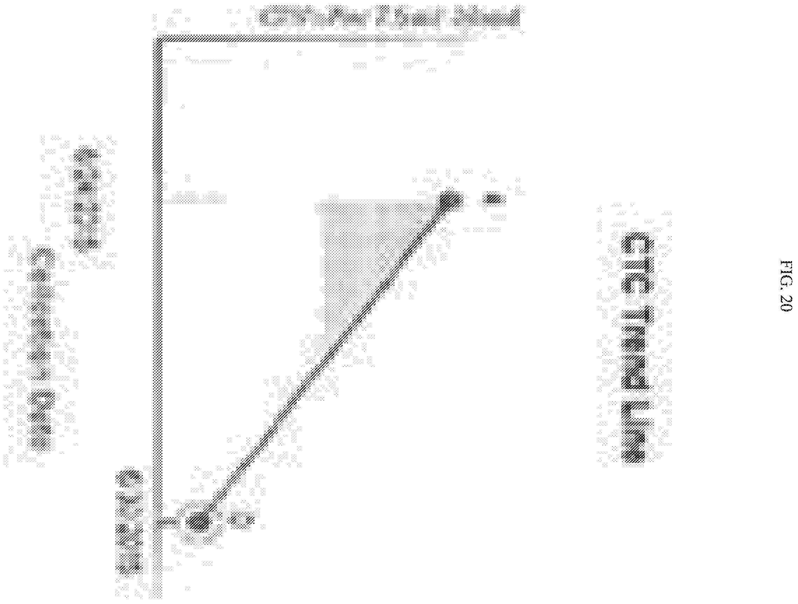

Her circulating tumor cell (CTC) assay was positive at 4 CTC per 7.5 ml of whole blood pre therapy. The CTC assay was repeated about one week post therapy and showed no CTC (FIG. 20). The clinical cut-off point for the CTC assay is <5.

Her circulatory DNA was repeated about 3 weeks after starting treatment and showed stable ERBB2 and P53, and the "mutated circulatory DNA of FBXW7" was no longer detectable in the blood, which was a marker indicative of response to the therapy. FBXW7 is an important marker for carcinogenesis and aggressiveness of the breast cancer and modulates C-MYC and Ki 67.

Her brain MRI and whole body PET scan were repeated about 6 weeks after starting treatment and showed significant partial resolution of previously seen extensive zones of FDG avid uptake on the skin surface of the right anterior, lateral, and posterior chest wall, moderate improvement in previously described FDG avid metastatic mediastinal, supraclavicular, and axillary lymph nodes, with only minimal residual disease remaining. Near complete resolution was observed of previously seen mid lung filed abnormalities. Brain MRI confirmed as well the decreased spectropgraphic findings in the left cerebellum.

She then returned to her home town in Italy. In summary, application of the disclosed combination therapy comprising, consisting of, or consisting essentially of, Temodar and Quercetin PG is feasible and clinically relevant. This study showed significant effectiveness in treating a patient with advanced cancer who had previously failed as many as 8 lines of chemotherapy. The results herein are very encouraging and provide impetus to implementing such an approach in advanced cancer.

### Example 18 - Case Study - Rebecca - Treatment of breast cancer with Quercetin PG-SPB, and Quercetin PG-Temodar

A 41 year old woman presented at the clinic of Dr. Nezami who had been diagnosed with metastatic and advanced ER-/PR-/Her-2+ left breast ductal adenocarcinoma about 5 years earlier. She was also found to have germline BRCA 2 mutation. At that time she underwent standard neoadjuvant chemotherapy (ACT regimen). Further she had undergone mastectomy.

Four years post diagnosis, PET scan revealed recurrence of her disease, with ER-/PR-/ Her-2+ disease (per pathology report from lung mass), and received treatment at Comprehensive Blood and Cancer Center. CT scan performed 9 months later (which was her most recent CT scan prior to at the clinic of Dr. Nezami) showed progression of disease with worsening metastasis in the bones (T7, T8, lumbar and sacral areas), multiple pulmonary nodules, and lymphadenopathy in the chest, bilateral massive pleural effusions, hepatomegaly and liver metastasis. She had been referred to hospice as she had failed therapies she had experimented. Her records from her oncologist showed declining function, (ECOG of 1-2) and worsening labs (LDH >1000, elevated LFTs, elevated tumor markers). Her oncologist had suggested hospice, which she had refused.

### Upon her arrival at the clinic of Dr. Nezami, she was evaluated both through liquid biopsy and tissue biopsy molecular profiling

Molecular detection of of circulating tumor cells was performed as follows. In order to obtain circulating tumor cells from the patient's peripheral blood, large cells and cell-clusters as well as epithelial cells were isolated. A preparation of mononuclear cells (MNC) served as a control cell fraction. mRNA was ioslated from all fractions. Thereafter, the expression of tumor relevant genes was measured by quantitative real-time RT-PCR. A preparation fo mononuclear cells (MNC) served as a control cell fraction.

The expression of the telomerase gene can be increaed in most tumor types but not in normal tissue. An increased expression of telomerase gene may be indicative of the presence of tumor cells in the circulation. Overexpression of telomerase was detected in the isoalted cells. Overexpression of C-MYC indicates an increased proliferation of the ioslated cells. An increased proliferation rate is a typical feature of tumor cells. The expression of level of C-MYC was not elevated. Overexpression of ERBB2 (HER-2/NEU) is a trait of different types of cancer and may also be observed in breast cancer. Thus, the detection of ERBB2 overexpression may be indicative of the preence of ciruclaitng tumor cells. The expression of ERBB2 was elevated. The detection of expression of cytokeratin (CK) 19 indicates the presence of epithelial cells and may thus be indicative for circulating tumor cells. Expression of CK19 was detected. Thus, in the isolated tumor cell fraction, expression of ERBB2 and telomerase was above threshold (>2.0) and expression of CK19 was above threshold (≥1) in the higher range (>1000). This finding was indicative of the likely presence of circulating tumor cells in the analyzed blood sample.

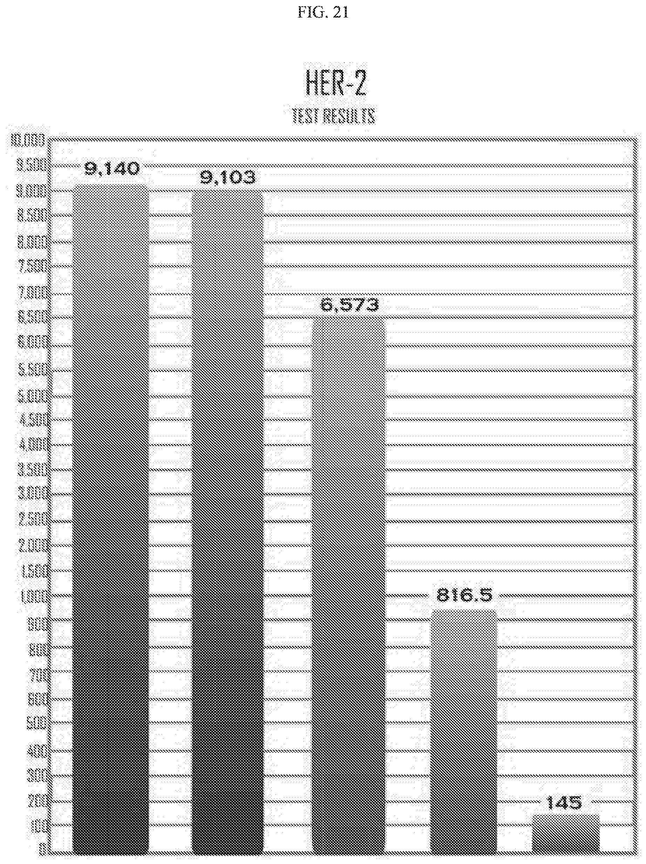

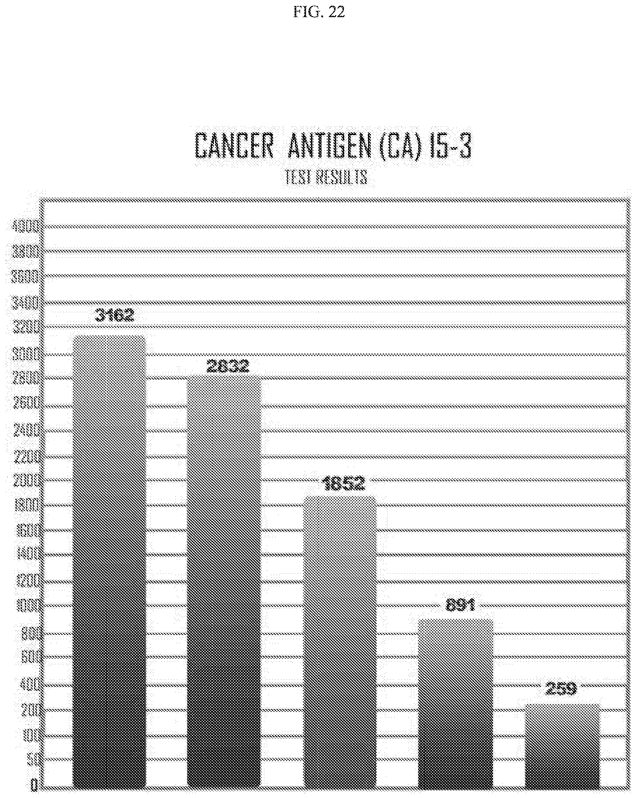

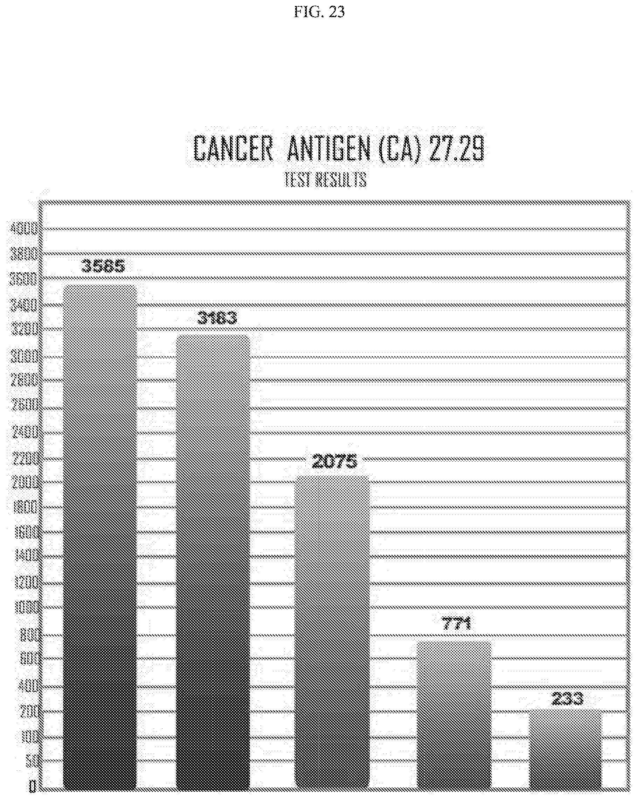

She also showed very elevated serum Her-2 level at 9140 at the start of treatment (FIG. 21). She had very high tumor markers as well (CA 15-3 was at 3162 (FIG. 22), and CA 27.29 was at 3585 (FIG. 23)). Additionally, her circulating DNA was positive for ERBB2, c-MYC, and TP53 mutations, and her CTC assay was positive for ERBB2 and CK19.

She was immediately started on IV epigenetic therapies, consisting of IV Quercetin PG as mainstay of her therapy along with SPB, on daily basis 5 times a week. Her quality of life quickly improved since starting the treatments. Her ECOG improved substantially with improved breathing and pain level. She was again able to walk independently and her jaundice resolved.

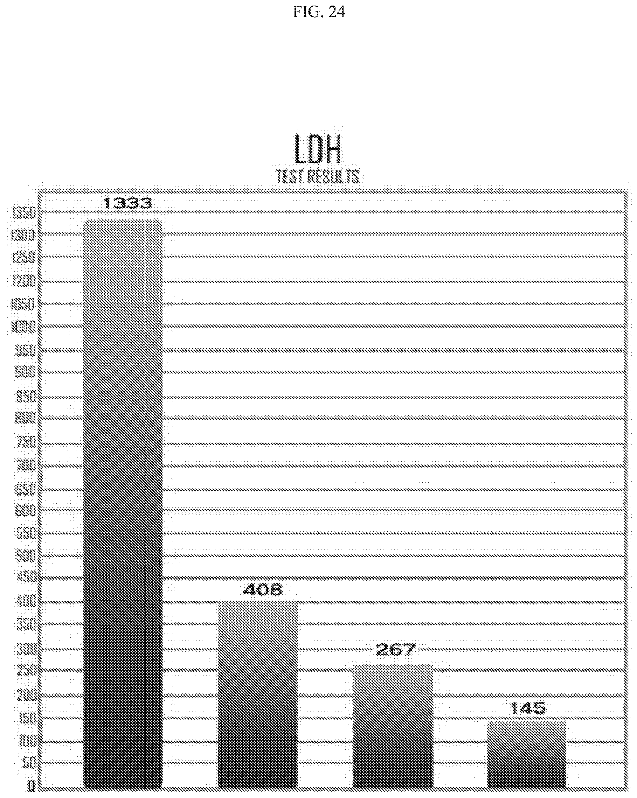

Her laboratory tests were repeated about 3 weeks later, which showed improved tumor markers, LDH and serum Her-2 levels. Her CA 15-3 decreased from 3162 to 2832 (FIG. 22), her CA 27.29 decreased from 3585 to 3183 (FIG. 23). Her serum Her-2 decreased from 9140 to 6573 measured about 3 weeks after beginning treatment (FIG. 21). Her LDH decreased to 408 from 1333 (FIG. 24). Her CTC also showed a marked reduction. These results were obtained after 13 treatments over the course of about 3 weeks.

Thereafter, her treatment frequency was decreased to twice a week with targeted/epigenetic therapies. Her laboratory tests were repeated 2 weeks later and, after only four treatments, showed marked reduction in all her markers. Her CA 15-3 decreased from 2832 to 1852 (FIG. 22), her CA 27.29 decreased from 3183 to 2075 (FIG. 23).

A restaging CT scan of chest was performed another 2 weeks later and showed partial response to interim therapy consisting of improvement in all pulmonary nodules as well as chest lymph nodes. There was substantial reduction in all lesions size noted. The density in the posterior right upper lobe of lung decreased in size from 41X16 mm to 27X8 mm, the density in the posterior left upper lobe decreased in size from 18X10 mm to 10X4 mm, the nodule in left medial upper lobe decreased from 12X6 mm to 7X4 mm, the nodule in the left perihilar lesion decreased from 19X14 mm to 11X10 mm, the lesion in posterior right lower lobe decreased from 20X17 mm to 15X12 mm, the left upper paratracheal node decreased from 7 to 3 mm, the left perivascular node decreased from 14X9 mm to poorly defined, and a peritracheal node decreased from 13X10 mm to 10X8 mm.

Her tumor markers further decreased again measured one day after her restaging CT scan. Her CA 15-3 decreased to 891 (FIG. 22), CA 27.29 decreased to 771 (FIG. 23), LDH decreased to 267 (FIG. 24), and her serum Her-2 decreased to 816.5 (FIG. 21).

A restaging PET scan was performed about another 7 weeks later showed all her pulmonary lesions, liver lesions and skeletal lesions responded to the therapy with metabolic activity of background and decreased sizes. For example, her right hepatic lobe lesion decreased from 58X43 mm to 42X33 mm. Another lesion that was 24X19 mm decreased to 18X16 mm. Nodular density in left upper lobe decreased to 8X5 mm from 10X4 mm. Her left pleural effusion was decreased in size. There was a necrotic mass in the right breast, and she demonstrated positive response to therapy with increasing sclerosis and healing in all skeletal bone metastasis.

Her tumor markers also continued to decrease. Serum Her-2 decreased to 145 (FIG. 21) as measured 3 days after her restaging PET from 816 as measured about 7 weeks earlier. Her CA 15-3 had decreased to 259 from 891 (FIG. 22), CA 27.29 had decreased to 233 from 771 (FIG. 23), and LDH had decreased to 159 from 267 (FIG. 24).

Unfortunately she was unable to continue the epigenetic therapies as scheduled due to her distant residence and developed brain metastasis about 5 weeks after her restaging PET with the largest metastasis in the cerebellum about 20 mm in size.

She was started on Temodar at 200 mg daily (1-5/28) in combination with IV Quercetin PG. She refused whole brain radiation and was consulted for gamma knife for her brain metastasis. However, she opted out of all radiotherapy options. She was further treated with two cycles of Temodar in conjunction with targeted therapies.

Further laboratory tests showed significant reduction of her tumor markers. Her Quality of Life improved and she was able to walk independently again. She has passed her expected survival with above measures of care.

In summary, application of the disclosed combination therapy comprising, consisting of, or consisting essentially of, Temodar and Quercetin PG is feasible and clinically relevant and showed significant effectiveness in treating metastatic and advanced breast adenocarcinoma.

### Example 19 - Case Study - Shannon - Treatment of breast cancer with Quercetin PG-SPB, and Quercetin PG-Temodar

This case study is of a 39 year old female patient with a history of stage four bilateral infiltrating moderately differentiated ductal carcinoma with angiolymphatic involvement, ER positive, PR negative, Her-2/Neu+3.

Diagnosis revealed involvement of the adrenals as determined by biopsies. The patient was subsequently started on Herceptin, carboplatinum and Taxotere of which she completed the six cycles resulting in complete remission.

Patient subsequently had mastectomy and radiation therapy followed by Herceptin for maintenance and hormonal blockade with tamoxifen/fareston. At this point, patient was in complete remission as determined by PET scan 7 months after completion of 6 cycles of treatment with Herceptin, carboplatinum and Taxotere.

Eleven months later patient developed seizures and MRI of brain showed five separate enhancing masses in the brain. The largest was resected immediately following detection and patient received whole brain radiation therapy.

Five months later patient relapsed with progression of disease with bone metastasis while she was on Tykerb and Xeloda. At this point, the patient started treatment at Cancer Treatment Centers of America and was prescribed Abraxane. The patient tried one session of Arbaxane, developed severe neuro- and hematological toxicity, and opted out of further chemotherapy. She continued to receive Herceptin and Xgeva, along with Faslodex (hormonal blockade) with progressive disease. Importantly, there were no further viable therapeutic options for her.

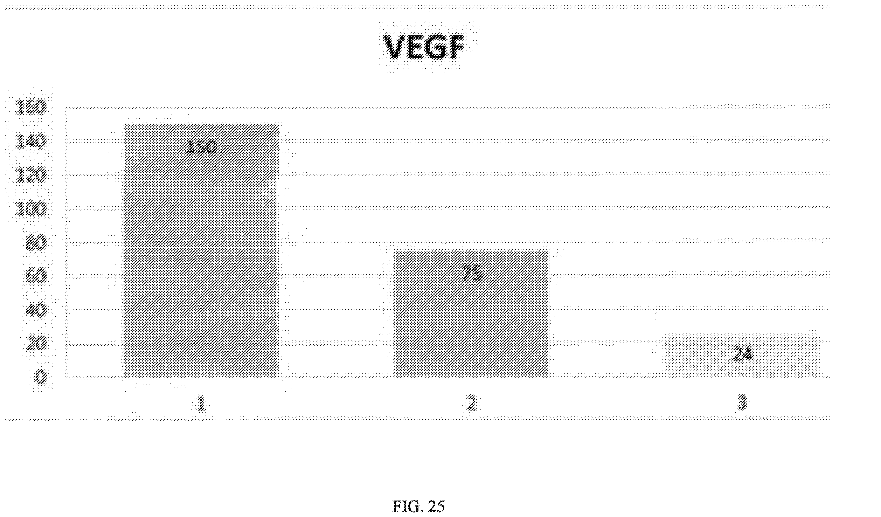

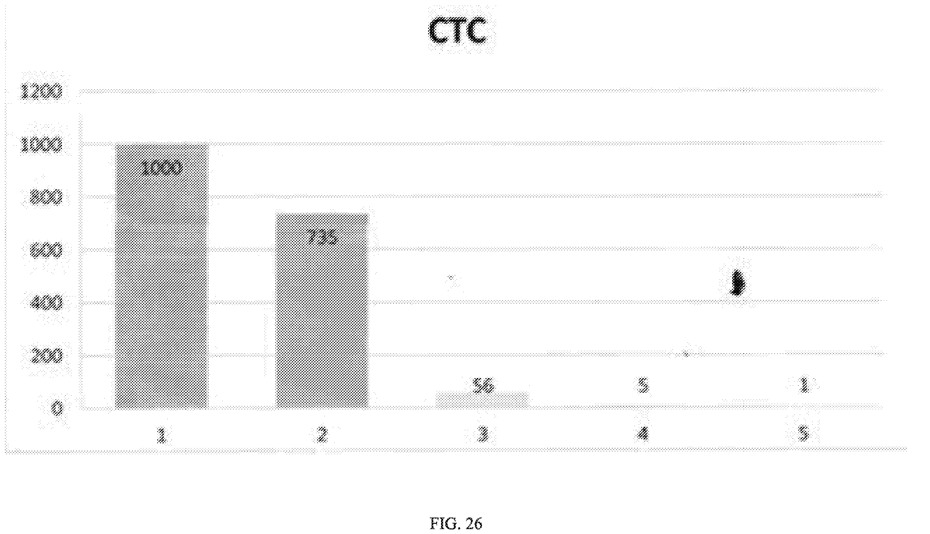

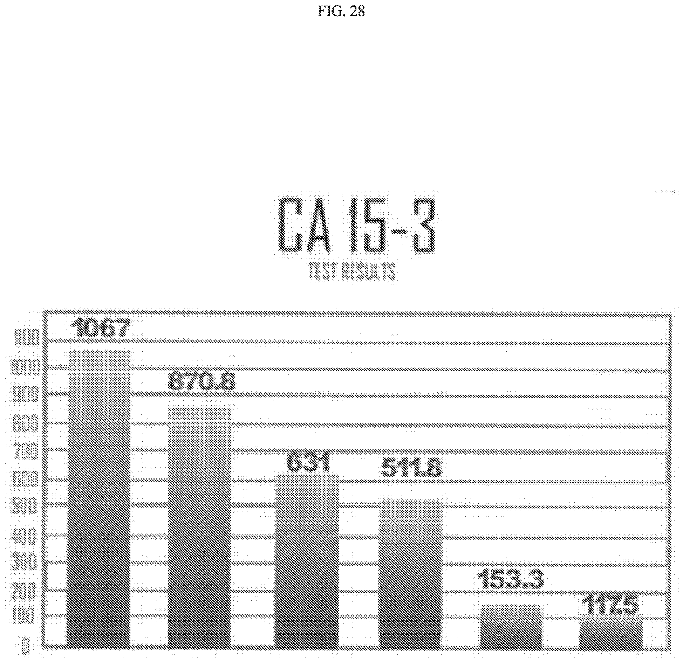

Thereafter, one year after her relapse, patient arrived at the clinic of Dr. Nezami as a last resort. Upon arrival her initial laboratory tests showed severe immune suppression due to suppressed NK activity, significantly elevated tumor markers (FIG. 28 - FIG. 30) and VEGF (FIG. 25), and circulatory tumor cells higher than 1000 per 7.5 ml of blood by Verdix (FIG. 26). It is noted that the maximum detectable limit for CTC is 1000! It is further noted that the threshold for a patient with metastatic breast cancer is 5 and a patient's chances of survival are poor if the number is above 5. In this case, the level was astronomically high at 1000!

She was immediately started at the clinic of Dr. Nezami on IV epigenetic treatments consisting of Quercetin PG, as mainstay of therapy and sodium phenyl butyrate (SPB) administered on daily basis, five times a week. She immediately started feeling better with increased energy.

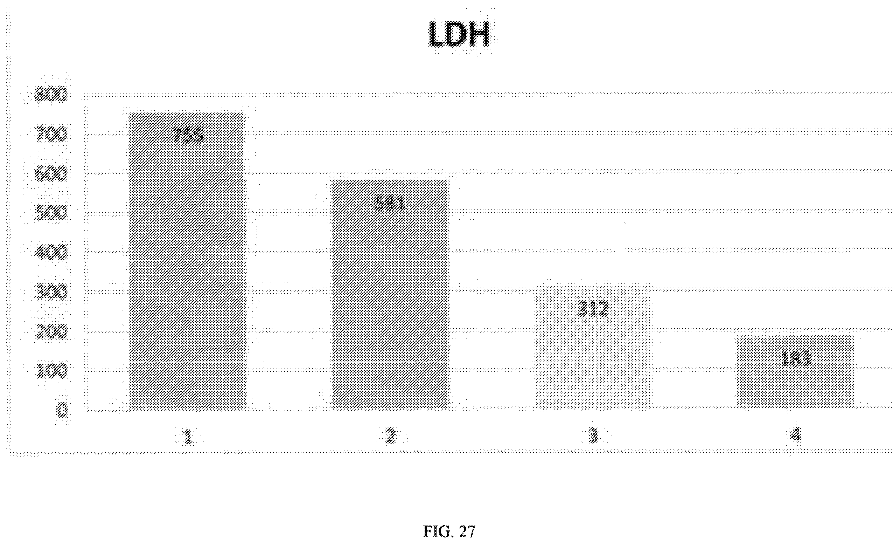

She did not experience any toxic side effects and her laboratory tests were repeated about 2 weeks later. Her VEGF decreased from 150 at the start of treatment to 75 about 2 weeks later (FIG. 25), and her CTC decreased from 1000 to 724 (FIG. 26) in the same time frame. Her LDH also decreases from 755 to 581 in the first two weeks after starting treatment, to 314 another two weeks later (FIG. 27), and normalized to 122 about 5 weeks. Her CEA also decreased from 388 to 327. Her VEGF decreased from 150 to 75 in just 14 days after receiving 7 treatments (FIG. 25). About 2 weeks thereafter, her CTC decreased again to 359 (after 20 treatments), and her VEGF decreased to from 150 to 24 (FIG. 25). Her quality of life and ECOG performance score improved from 2 to zero (i.e., fully active).

A whole body scan and brain MRI were repeated, about 2 months after starting treatment, which revealed no lesions in the MRI (normal), and resolved pulmonary findings in her PET scan with only remnant bony lesions and stable disease. After she had received 47 treatments, her CTC was repeated (about one month after whole body scan and brain MRI), which showed a decrease from 1000 to 56 (FIG. 26). Her VEGF stayed normal at 112 (as measured about 2 weeks after the repeat CTC assay) even after tapering down the treatments to once a week.

Her treatments were continued on twice weekly basis, and her markers continued to decrease over a 3 month period. Her CEA decreased from 926 when the whole body scan and brain MRI were repeated (February 2013) to 239 and further to 194. During this time, her LDH decreased from 504 to 241 and 183, and her CA 15-3 decreased from 684 to 311 and then to 294, her CA 27.29 decreased from 881 to 290.5 as measured after 1 month, and 285 as measured after 3 months. Another analysis about 2 weeks later showed that the markers had further decreased - CEA had decreased to 182, CA 15-3 to 232, and CA 27.29 to 258.

Her CTC was repeated after about 17 weeks of treatment and had decreased from 1000 prior to initiating treatment to 5 post treatment (FIG. 26). After another 5 weeks a repeat CTC assay showed that the level had decreased to just 1 (FIG. 26), which further decrease to zero.

Her PET scan was repeated for restaging about 1 week later, which showed physiologic activities in the chest and no metabolic activity at known bone metastatic lesions observed in prior exams. There was no marrow involvement noted compared to prior MRI findings of marrow enhancement. She had been on twice weekly treatment for about 70 days at this point - the frequency of treatments was subject to patient's availability for administration of treatment.

As she was experiencing of neck pain, about 1 month later her oncologist referred her for possible spinal involvement by the tumor. The pathology of a herniated disc showed no evidence of malignancy.

Additionally, a PET scan revealed a pulmonary nodule and suspicious lymphangiomatosis. Therefore, she was referred for bronchoscopy and the pathology was negative for malignancy in all specimens. As such she was found to have no residual disease.

About 2 months later, she received one injection of TDM-1. However, her tumor markers did not decrease and she experienced significant toxicity. Indeed, the markers increased when compared to levels from about 1 month ago after receiving the TDM-1. Her CA 15-3 increased from 695 to 1062, CA 27.29 increased from 763 to 1313, and her CEA increased from 669 to 972. As a result, she decided not to receive further chemotherapy.

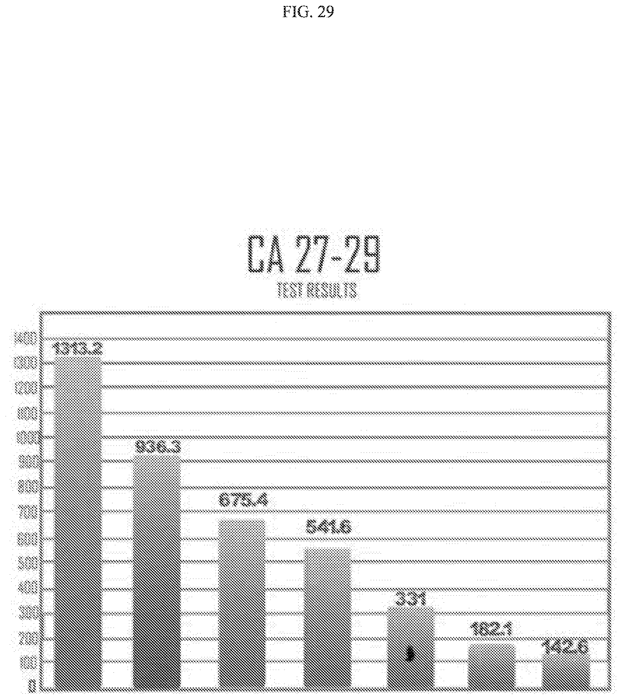

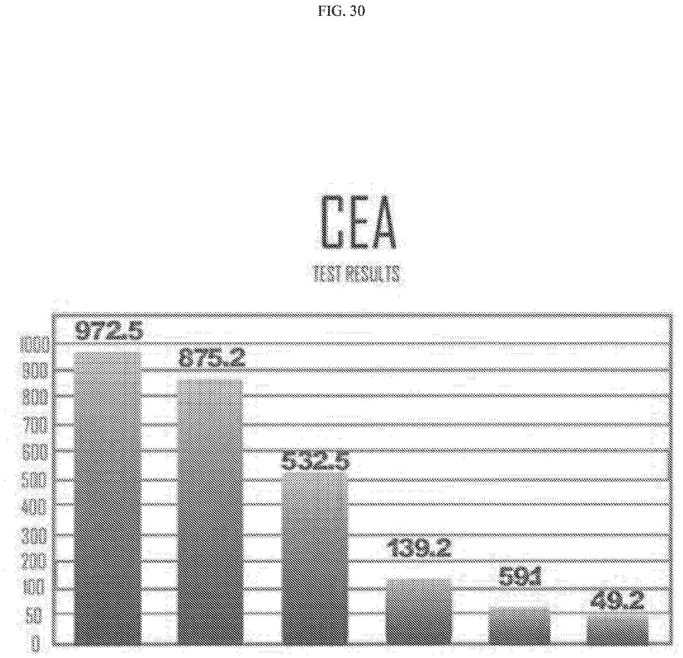

She continued to receive IV epigenetic therapies on biweekly basis at the clinic of Dr. Nezami. About 1 month later, her markers decreased again - CEA decreased from 972 to 532 (FIG. 30), CA 15-3 decreased from 1067 to 631 (FIG. 28), and CA 27.29 decreased from 1313 to 674 (FIG. 29). She received no hormonal blockade or other therapies (Xgeva, bisphosphonates) during this time.

Further, for optimal control of her CNS disease, she received monthly Doxil injections along with epigenetic therapies in combination. Her tumor markers continued to decrease further as she continued treatment at the clinic of Dr. Nezami. In about 4 weeks after starting treatment, her CEA decreased from 972 to 532 (FIG. 30), CA 15-3 decreased from 1067 to 511 (FIG. 28), and CA 27.29 decreased from 1313 to 541 (FIG. 29).

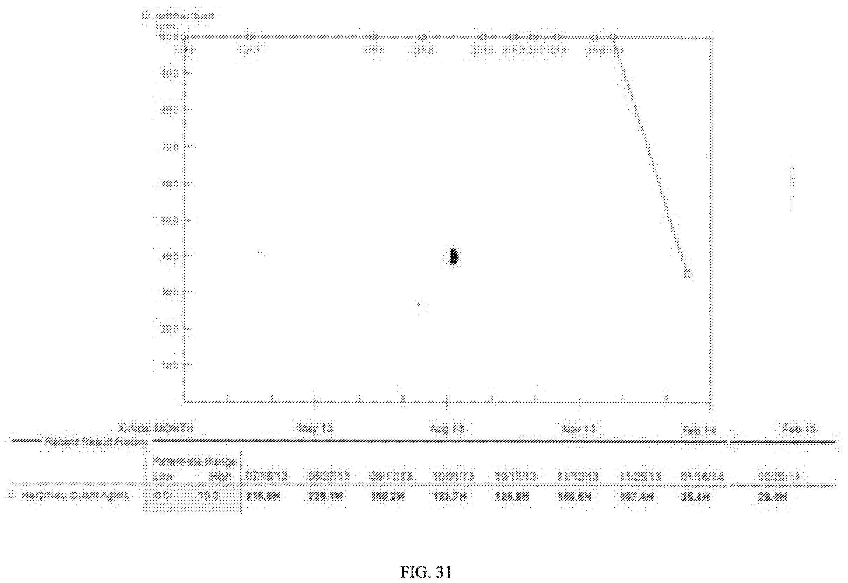

Her LDH had decreased from 543 to 262, and Her-2 had decreased from 225 to 108 (FIG. 31) (about 50 percent reduction in less than 3 weeks). A repeat brain MRI was performed about 3 months later, which revealed overall decreased in size of the brain metastatic lesions in both cerebellum and the parenchyma, compared on the prior MRI about 2 months ago. The largest lesion at the site of suboccipital craniotomy had also reduced in size.

For about 6 months she had been receiving Temodar on daily schedule (1-5/28) in combination with IV epigenetic therapies, with extremely positive results. Her PET scan around the time of her repeat brain MRI also confirmed partial response and shrinkage of tumor in all metastatic sites. There were significant improvement in level of activity of all lesions in brain, as well as bones (complete resolution/response) and thyroid, left mid iliac bone lesion, right posterior element of L4, S1 joint lesion, adrenal gland lesion, previously seen mass, left periaortic node activity, thyroid mass, pulmonary large fissure, pleural space effusions, all showed complete resolution, and left cerebellum was less intense and smaller. Her SUV had decreased from 14 to 9.

Additionally, all her markers had decreased - CA 15-3 decreased to 153.3 (FIG. 28), CA 27.29 to 182.1 (FIG. 29), and CEA to 59.1 (FIG. 30) as measured about 1 month after her PET scan. Her VEGF also continued to stay in normal range around 105 and Her-2 levels had decreased from 225 to 35 over the course of about 5 months (FIG. 31). Her CTC was also measured around this time had decreased to zero as compared to 1000 about 14 months ago.

In summary, such unexpected and impressive response in a patient with advanced cancer following several unsuccessful attempts with a variety of conventional therapies warrants further investigation and can significantly impact the current standards of care in oncology.

### Example 20 - Cell lines and maintenance, preparation of modulators, MiCK^{®} assay and data analysis

Examples of cells include AU-565 (breast carcinoma), MCF-7 (breast adenocarcinoma); MDA-MB-468 (breast adenocarcinoma), NCI-H23 (lung adenocarcinoma), NCI-H460 (non-small cell lung carcinoma), NCI-H1299 (non-small cell lung carcinoma), BxPC-3 (pancreatic adenocarcinoma), MIA PaCa-2 (pancreatic adenocarcinoma), Malme-3M (malignant melanoma), A-172 (glioblastoma), MES-SA (uterine sarcoma). These cell lines are already resistant to one or more anti-cancer agents.

The cells lines are grown and maintained in their specific cell culture medium without phenol red, supplemented with 10% FBS, 100 U/mL penicillin and 100 µg/mL streptomycin in humidified air with 5% CO₂ at 37°C. Before use, exponentially growing cells are harvested, washed with pre-warmed medium and then re-suspended in complete medium.

A stock solution of quercetin is prepared in 20% DMSO at a concentration of 500 µM and then diluted to 100 µM in the culture medium. The final concentration of DMSO in the well is 1% or less. A slight yellow color is encountered but the color is not considered significant in the assay. Sodium phenyl butyrate is dissolved in RPMI at a concentration of 20 µM without an interfering color development. Epigallocatechin-3-gallate is dissolved in RPMI at a concentration of 10 µg/mL.

The MicroCulture^{®} Kinetic (MiCK^{®}) assay is used to detect and quantitate apoptotic cell death in a population of cells. The cells of interest are exposed to the drug(s) of interest in the wells of a 384 well spectrophotometric plate with RPMI or DMEM as a support medium. Changes in cell shape are detected by automated spectrophotometric readings automatically recorded every five minutes.

The optical density (OD) readings obtained in the MiCK^{®} assay are plotted versus time to obtain a curve. The slope of the obtained curve is used in a proprietary equation to determine the amount of apoptosis induced. The unit of measure is the kinetic unit (KU). Although the disclosure refers to tests occurring in the wells of a 384 well spectrophotometric plate, one of skill in the art will recognize that numerous test sites are suitable for the test disclosed herein, and therefore, a 384 well spectrophotometric plate is a not limiting embodiment. For example, spectrophotometric plates of other sizes (6, 12, 24, 48, 96 well plates), cuvettes, test tubes, or any other suitable structure known in the art can be used.

### Abbreviations

NPs Nanoparticles
QC Quercetin
FITC Fluorescein isothiocyanate
PLGA poly (lactide-*co*-glycolide)
PEG Polyethylene Glycol
FA Folic acid
DSPE_PEG 1,2-Distearoyl-*sn*-Glycero-3-Phosphoethanolamine-Amino (Polyethylene Glycol)
DLS Dynamic Light Scattering
NHS N-Hydroxysuccinimide

## Claims

1. A pharmaceutical composition for use in prophylaxis, treatment or both of a neoplasm, wherein the pharmaceutical composition comprises a nanoparticle formulation comprising quercetin, and at least one anti-cancer agent, wherein the at least one anti-cancer agent is temozolomide (Temodar)

2. The pharmaceutical composition for use according to claim 1, wherein the pharmaceutical composition is formulated for IV administration or oral administration.

3. The pharmaceutical composition for use according to any one of the preceding claims, wherein:
a. the amount of quercetin is 0.1 g to 2.5 g; and/or
b. quercetin is in solution at a concentration of 10 mg/ml to 500 mg/ml.

4. The pharmaceutical composition for use according to any one of the preceding claims, wherein the amount of Temodar ranges between 20 mg and 5000 mg.

5. The pharmaceutical composition for use according to any one of the preceding claims, wherein:
c. the at least one anti-cancer agent and quercetin are in a single dosage form for co-administration;
d. the at least one anti-cancer agent and quercetin are in a single dosage form suitable for IV administration;
e. the at least one anti-cancer agent and quercetin are in a single dosage form suitable for oral administration;
f. the anti-cancer agent and quercetin are in a separate dosage forms;
g. anti-cancer agent and quercetin are each in dosage forms suitable for IV administration;
h. the anti-cancer agent and quercetin are each in dosage forms suitable for oral administration; or
i. the anti-cancer agent or quercetin is in a dosage form suitable for oral administration and the other is in a dosage form for IV administration.

6. The pharmaceutical composition for use according to any one of the preceding claims, wherein the neoplasm is one or more of astrocytomas, pilocytic astrocytoma, diffuse astrocytoma, anaplastic astrocytoma, and brain stem gliomas, glioblastomas, glioblastomas multiforme, meningioma, gliomas, ependymomas, oligodendrogliomas, and mixed gliomas, brain tumors, pituitary tumors, craniopharyngiomas, germ cell tumors, pineal region tumors, medulloblastomas, and primary CNS lymphomas.

7. A kit for use in prophylaxis, treatment or both of a neoplasm, wherein the kit comprises a pharmaceutical composition according to any one of claims 2-6, wherein each of the at least one anti-cancer agent and quercetin are contained in separate sub-containers.

8. The pharmaceutical composition or the kit for use according to any one of the preceding claims for treatment, prevention or both of a neoplasm in a subject in need thereof.

9. The pharmaceutical composition or the kit for use according to claim 8, wherein:
j. the neoplasm is likely to develop resistance, develops resistance, and/or is already resistant to the at least one anti-cancer agent; or
k. the neoplasm is likely to develop resistance, develops resistance, and/or is already resistant to quercetin.

10. The pharmaceutical composition or the kit for use according to any one of the preceding claims, wherein the composition induces apoptosis in vitro in at least one cancer cell line.

11. The pharmaceutical composition or the kit for use according to claim 10, wherein:
l. the induction of apoptosis by the composition is additive as compared to the induction of apoptosis of each of the anti-cancer agents and quercetin alone; or
m. the induction of apoptosis by the composition is synergistic as compared to the induction of apoptosis of each of the anti-cancer agents and quercetin alone.

12. The pharmaceutical composition or the kit for use according to any one of the preceding claims wherein a dimension of the nanoparticle in the nanoparticle formulation ranges from about 100 nm to about 250 nm.

13. The pharmaceutical composition or kit for use according to any one of the preceding claims wherein the nanoparticle is selected from the group consisting of PLGA-PEG-NPs, FA-PLGA-PEG-NPs, DSPE-PEG-NPs and FA-DSPE-PEG-NPs.

## Patentansprüche

1. Pharmazeutische Zusammensetzung zur Verwendung bei der Prophylaxe, Behandlung oder beidem eines Neoplasmas, wobei die pharmazeutische Zusammensetzung eine Nanopartikelformulierung umfassend Quercetin und mindestens ein Antikrebsmittel umfasst, wobei das mindestens eine Antikrebsmittel Temozolomid (Temodar) ist.

2. Pharmazeutische Zusammensetzung zur Verwendung nach Anspruch 1, wobei die pharmazeutische Zusammensetzung zur intravenösen Verabreichung oder oralen Verabreichung formuliert ist.

3. Pharmazeutische Zusammensetzung zur Verwendung nach einem der vorhergehenden Ansprüche, wobei:
a. die Menge an Quercetin 0,1 g bis 2,5 g beträgt; und/oder
b. Quercetin in einer Konzentration von 10 mg/ml bis 500 mg/ml in Lösung vorliegt.

4. Pharmazeutische Zusammensetzung zur Verwendung nach einem der vorhergehenden Ansprüche, wobei die Menge an Temodar im Bereich zwischen 20 mg und 5000 mg liegt.

5. Pharmazeutische Zusammensetzung zur Verwendung nach einem der vorhergehenden Ansprüche, wobei:
c. das mindestens eine Antikrebsmittel und Quercetin in einer Einzeldosierungsform zur gleichzeitigen Verabreichung vorliegen;
d. das mindestens eine Antikrebsmittel und Quercetin in einer Einzeldosierungsform vorliegen, die für die intravenöse Verabreichung geeignet ist;
e. das mindestens eine Antikrebsmittel und Quercetin in einer Einzeldosierungsform vorliegen, die für die orale Verabreichung geeignet ist;
f. das Antikrebsmittel und Quercetin in separaten Dosierungsformen vorliegen;
g. das Antikrebsmittel und Quercetin jeweils in Dosierungsformen vorliegen, die für die intravenöse Verabreichung geeignet sind;
h. das Antikrebsmittel und Quercetin jeweils in Dosierungsformen vorliegen, die für die orale Verabreichung geeignet sind; oder
i. das Antikrebsmittel oder Quercetin in einer Dosierungsform vorliegt, die für die orale Verabreichung geeignet ist, und das andere in einer Dosierungsform für die intravenöse Verabreichung vorliegt.

6. Pharmazeutische Zusammensetzung zur Verwendung nach einem der vorhergehenden Ansprüche, wobei das Neoplasma eines oder mehrere von Astrozytomen, pilozytischem Astrozytom, diffusem Astrozytom, anaplastischem Astrozytom und Hirnstammgliomen, Glioblastomen, Glioblastomas multiformes, Meningeomen, Gliomen, Ependymomen, Oligodendrogliomen und gemischten Gliomen, Hirntumoren, Hypophysentumoren, Kraniopharyngeomen, Keimzelltumoren, Tumoren der Zirbeldrüsenregion, Medulloblastomen und primären ZNS-Lymphomen ist.

7. Kit zur Verwendung bei der Prophylaxe, Behandlung oder beidem eines Neoplasmas, wobei der Kit eine pharmazeutische Zusammensetzung nach einem der Ansprüche 2-6 umfasst, wobei jedes von dem mindestens einen Antikrebsmittel und Quercetin in separaten Unterbehältern enthalten ist.

8. Pharmazeutische Zusammensetzung oder Kit zur Verwendung nach einem der vorhergehenden Ansprüche zur Behandlung, Vorbeugung oder beidem eines Neoplasmas bei einem Subjekt, das dessen bedarf.

9. Pharmazeutische Zusammensetzung oder Kit zur Verwendung nach Anspruch 8, wobei:
j. das Neoplasma gegen das mindestens eine Antikrebsmittel wahrscheinlich eine Resistenz entwickeln wird, eine Resistenz entwickelt und/oder bereits resistent dagegen ist; oder
k. das Neoplasma gegen Quercetin wahrscheinlich eine Resistenz entwickeln wird, eine Resistenz entwickelt und/oder bereits resistent dagegen ist.

10. Pharmazeutische Zusammensetzung oder Kit zur Verwendung nach einem der vorhergehenden Ansprüche, wobei die Zusammensetzung in vitro Apoptose in mindestens einer Krebszelllinie induziert.

11. Pharmazeutische Zusammensetzung oder Kit zur Verwendung nach Anspruch 10, wobei:
l. die Induktion von Apoptose durch die Zusammensetzung im Vergleich zur Induktion von Apoptose durch jedes der Antikrebsmittel und Quercetin allein additiv ist; oder
m. die Induktion von Apoptose durch die Zusammensetzung im Vergleich zur Induktion von Apoptose durch jedes der Antikrebsmittel und Quercetin allein synergistisch ist.

12. Pharmazeutische Zusammensetzung oder Kit zur Verwendung nach einem der vorhergehenden Ansprüche, wobei eine Abmessung des Nanopartikels in der Nanopartikelformulierung von etwa 100 nm bis etwa 250 nm reicht.

13. Pharmazeutische Zusammensetzung oder Kit zur Verwendung nach einem der vorhergehenden Ansprüche, wobei das Nanopartikel ausgewählt ist aus der Gruppe bestehend aus PLGA-PEG-NPs, FA-PLGA-PEG-NPs, DSPE-PEG-NPs und FA-DSPE-PEG-NPs.

## Revendications

1. Composition pharmaceutique à utiliser dans la prophylaxie, le traitement ou les deux d'un néoplasme, la composition pharmaceutique comprenant une formulation de nanoparticules comprenant la quercétine et au moins un agent anticancéreux, ledit au moins un agent anticancéreux étant le témozolomide (Temodar).

2. Composition pharmaceutique à utiliser selon la revendication 1, dans laquelle la composition pharmaceutique est formulée pour une administration IV ou une administration orale.

3. Composition pharmaceutique à utiliser selon l'une quelconque des revendications précédentes, dans laquelle :
a. la quantité de quercétine est de 0,1 g à 2,5 g ; et/ou
b. la quercétine est en solution à une concentration de 10 mg/ml à 500 mg/ml.

4. Composition pharmaceutique à utiliser selon l'une quelconque des revendications précédentes, dans laquelle la quantité de Temodar est comprise entre 20 mg et 5 000 mg.

5. Composition pharmaceutique à utiliser selon l'une quelconque des revendications précédentes, dans laquelle :
c. l'au moins un agent anticancéreux et la quercétine sont sous une forme posologique unique pour une co-administration ;
d. l'au moins un agent anticancéreux et la quercétine sont sous une forme posologique unique adaptée à l'administration IV ;
e. l'au moins un agent anticancéreux et la quercétine sont sous une forme posologique unique adaptée à l'administration orale ;
f. l'agent anticancéreux et la quercétine sont sous des formes posologiques distinctes ;
g. l'agent anticancéreux et la quercétine sont chacun sous des formes posologiques adaptées à l'administration IV ;
h. l'agent anticancéreux et la quercétine sont chacun sous des formes posologiques adaptées à l'administration orale ; ou
i. l'agent anticancéreux ou la quercétine est sous une forme posologique adaptée à l'administration orale et l'autre est sous une forme posologique adaptée à l'administration IV.

6. Composition pharmaceutique à utiliser selon l'une quelconque des revendications précédentes, dans laquelle le néoplasme est un ou plusieurs parmi les astrocytomes, l'astrocytome pilocytique, l'astrocytome diffus, l'astrocytome anaplasique, et les gliomes du tronc cérébral, les glioblastomes, les glioblastomes multiforme, le méningiome, les gliomes, l'épendymomes, les oligodendrogliomes, et les gliomes mixtes, les tumeurs cérébrales, les tumeurs hypophysaires, les craniopharyngiomes, les tumeurs germinales, les tumeurs de la région pinéale, les médulloblastomes, et les lymphomes primaires du SNC.

7. Kit à utiliser dans la prophylaxie, le traitement ou les deux d'un néoplasme, le kit comprenant une composition pharmaceutique selon l'une quelconque des revendications 2 à 6, dans laquelle chacun dudit au moins un agent anticancéreux et la quercétine sont contenus dans des sous-conteneurs séparés.

8. Composition pharmaceutique ou kit à utiliser selon l'une quelconque des revendications précédentes pour le traitement, la prévention ou les deux d'un néoplasme chez un sujet en ayant besoin.

9. Composition pharmaceutique ou kit à utiliser selon la revendication 8, dans lequel :
j. le néoplasme est susceptible de développer une résistance, développe une résistance et/ou est déjà résistant à au moins un agent anticancéreux ; ou
k. le néoplasme est susceptible de développer une résistance, développe une résistance et/ou est déjà résistant à la quercétine.

10. Composition pharmaceutique ou kit à utiliser selon l'une quelconque des revendications précédentes, dans lequel la composition induit l'apoptose in vitro dans au moins une lignée cellulaire cancéreuse.

11. Composition pharmaceutique ou kit à utiliser selon la revendication 10, dans lequel :
l. l'induction de l'apoptose par la composition est additive par rapport à l'induction de l'apoptose de chacun des agents anticancéreux et de la quercétine seul ; ou
m. l'induction de l'apoptose par la composition est synergique par rapport à l'induction de l'apoptose de chacun des agents anticancéreux et de la quercétine seul.

12. Composition pharmaceutique ou kit à utiliser selon l'une quelconque des revendications précédentes, dans lequel une dimension de la nanoparticule dans la formulation de nanoparticules varie d'environ 100 nm à environ 250 nm.

13. Composition pharmaceutique ou kit à utiliser selon l'une quelconque des revendications précédentes, dans lequel la nanoparticule est choisie dans le groupe constitué de PLGA-PEG-NP, FA-PLGA-PEG-NP, DSPE-PEG-NP et FA-DSPE-PEG-NP.
